# EUROPEAN PATENT SPECIFICATION

(11) **EP 0 417 524 B1**
(45) Date of publication and mention of the grant of the patent: **03.05.1995**
(21) Application number: 90116165.3
(22) Date of filing: 23.08.1990
(51) Int. Cl.: C12N 15/30, A61K 39/018, C07K 1/00, C12P 21/02, C12P 21/08, C12N 15/62, G01N 33/569, G01N 33/577, C12Q 1/68

(54) **Antigens and polypeptides derived from babesia (12D3 antigen)**
Von Babesia abgeleitete Antigene und Polypeptide (12D3-Antigen)
Antigènes et polypeptides dérivés de Babesia (antigène 12D3)

(30) Priority: 23.08.1989 AU 5902/89
(43) Date of publication of application: 20.03.1991
(73) Proprietor: COMMONWEALTH SCIENTIFIC AND INDUSTRIAL RESEARCH ORGANISATION, Campbell, ACT 2601 (AU)
(72) Inventor: Riddles, Peter William, Sunnybank Hills, Queensland 4109 (AU); Aylward, Jamese Harrison, St. Lucia, Queensland 4067 (AU); Wright, Ian Gordon, Sherwood, Queensland 4075 (AU)
(74) Representative: Hansen, Bernd, Dr. Dipl.-Chem.

(56) References cited:
- EP-A- 0 382 012
- WO-A-84/01897
- INFECTION AND IMMUNITY, vol. 56, no. 9, September 1988, pages 2363-2368, Washington, DC, US; W. GOFF et al.: "Identification of Babesia bovis merozoite surface antigens by using immune bovine sera and monoclonal antibodies"
- JOURNAL OF IMMUNOLOGY, vol. 138, no. 7, 1st April 1987, pages 2298-2304, Baltimore, US; T. McELWAIN et al.: "Antibodies define multiple proteins with epitopes exposed on the surface of live Babesia bigemina merozoites"

## Description

This invention relates to DNA sequences, recombinant DNA molecules and processes for producing polypeptides or antigens eliciting antibodies protective against babesiosis when administered to suitable domestic animals including cattle. The invention also relates to such polypeptides and antigens as well as vaccines produced from these polypeptides or related polypeptides or antigens which vaccines are highly protective against Babesia infection, or infection with any other parasite species.

More particularly, the invention relates to DNA sequences expressed in appropriate host organisms. The recombinant DNA molecules disclosed herein are characterised by DNA sequences that code for polypeptides which have the immunological activity of a polypeptide of B. bovis or any other species. A particular example of such a polypeptide is designated as antigen 12D3.

Antigen 12D3 is an antigen reactive with the monoclonal antibody secreted by an associated hybridoma MAbA12D3, which hybridoma was deposited with the European Collection of Animal Cell Cultures on 28th July 1989 under accession number 89072701 as A12D3.

Accordingly, this invention further relates to the above hybridoma, the monoclonal antibody secreted by the hybridoma and antigens specifically reactive with this monoclonal antibody.

However it will be appreciated that the invention includes within its scope any monoclonal antibody derived from antigens related to antigen 12D3 eliciting antibodies protective against babesiosis and that therefore the invention is not restricted to the monoclonal antibody secreted by the above hybridoma. The invention also includes within its scope any hybridoma capable of producing the abovesaid monoclonal antibodies exemplified by the hybridoma MAbA12D3.

Babesia is an intraerythrocytic parasite and is the major causative agent of babesiosis or red-water fever in domestic cattle in tick infested areas including tropical and subtropical pastures. Cattle that are moved from tick free to tick infested areas are particularly susceptible. Early work on the immunity conferred on infected animals which survived indicated that the immune response was humoral, since immunity was conferred to calves by colostrum immunoglobulins from infected mothers.

Unresolved antibodies from animals immune to B. bovis were subsequently shown to elicit immunity when transferred to non-immune animals.

Hitherto vaccines have been produced which protect cattle against the severe clinical manifestations of B. bovis parasitaemia. The vaccines used date include those using killed B. bovis, live vaccines using B. bovis attenuated by either irradiation or rapid passage in splenectomised calves and vaccines derived from in vitro culture supernatant antigens. Although such vaccines are to some extent protective, they all have the inherent problem of containing many antigens. Vaccination with such vaccines therefore elicits an immune response which places the immune system of the vaccinated animal under considerable stress. Also, multi-antigenic vaccines may contain antigens which elicit a strong response activity but which are not especially protective due to the location or chemistry of the antigen in the virulent field strains. This strong response may mask or otherwise inhibit the development of immunity based on antibody response to the presence of a less reactive antigen capable in isolation of conferring protective immunity. Where live attenuated vaccines are used, contamination of the vaccine with other pathogens may occur. Animals vaccinated with the live vaccines are subclinically infected carriers of the parasite and the potential for breakthrough infection due to de-attenuation is present. Refrigerated vaccine has a shelf life of only seven days.
Live vaccines in current usage also have a number of obvious limitations such as difficulty in transportation and possible reversion for the attenuated strain to a virulent strain. A killed vaccine consisting of well defined components would have a greater degree of acceptability and an obvious market.

In order to identify specific babesial antigens which confer protective immunity, babesial proteins from infected erythrocytes have been fractionated and assayed for immunoprotective potential. Protective antigens have been found in the soluble protein fraction obtained from lysis of babesia-infected erythrocytes as described in Australian Patent Specification Mo. 553779. Monoclonal antibodies have been raised against the protective soluble babesia protein fractions, and IFA, ELISA and Western blotting techniques used to identify clones producing monoclonal antibodies against specific babesial antigens. Antigens were then affinity purified with the monoclonal antibodies to yield single antigens for vaccination testing.

The above is also discussed in an article entitled "Protective Vaccination against Virulent B. bovis with a Low Molecular Weight Antigen" by Wright et al Infection and Immunity **48** 109-113 as well as an article by Wright et al in Infection and Immunity **41**, 244-250 and an abstract published in J. Cellular Biochemistry (1986) concerning the UCLA Symposia on Molecular and Cellular Biology.

Whilst purified antigen may be produced from babesial lysates by using the monoclonal antibody to that antigen, this method is not suitable for large scale production of vaccination grade product. The requirement therefore arises for improved commercial scale processes for the manufacture of antigen and polypeptide having like immunogenic activity.

Attention has therefore been focused on the use of recombinant DNA techniques to transform non-babesial hosts with babesial genes.

Kemp et al (Mol. Biochem. Parasit., 12 (1984) 61 - 67) discloses that Babesia genes can transform the eukaryotic host Saccharomyces cerevisiae, and that the host produces poly A⁺ RNAs corresponding to the transforming genes. However, there is no data to indicate that the polypeptides corresponding to the poly A⁺ mRNA obtained from K-strain B. bovis-infected erythrocytes were protective. The library was amplified in λgt10, cleaved, radiolabelled, fractionated, and religated into an ampicillin-resistance- conferring expression vector λ-amp3. The phage were packaged in vitro and plated out on E. coli BTA282 on ampicillin-containing media, and colonies producing cDNA detected by colony hybridisation. Colonies expressing Babesia antigens were detected by autoradiography using bovine antiserum to Ka strain B. bovis and ¹25I-labelled antibovine Ig. One clone was selected for further study (designated KaBb1). This clone produced a fusion protein of β-galactosidase and a babesial antigen with a Mᵣ 5000-10000 larger than native β-galactosidase.

Anti-Ka sera were fractionated against the fusion protein to yield affinity-purified anti-KaBb1 antibodies. These antibodies were used to detect an antigen with a Mᵣ of 220,000, corresponding to the dominant Mᵣ 220,000 antigen detected by unfractionated serum. Immunofluorescent assay indicated that anti-KABb1 reacts only with the Babesia parasite and not with the surrounding erythrocytic cytoplasm in vitro. By contrast, unfractionated anti-Ka serum reacts severally with the infected erythrocyte cytoplasm and the parasite.

However, vaccines formulate from the KaBb1/β-galactosidase fusion protein did not exhibit significant protective immunisation of animals against virulent heterologous B. bovis challenge (Timms et al Res Vet Sci). Indeed, the results indicated that the KaBb1/β-galactosidase vaccines were less efficacious than live Ka vaccines.
Reference also may be made to Cowman described in Manipulation and Expression of Genes In Eukaryotes (1983), 185-188 wherein a gene coding for avirulence associated polypeptides of B. bovis were isolated by differential colony hybridisation.

Poly A⁺ RNA was isolated from K-avirulent (Ka) strain by extraction from parasitised cattle erythrocytes and oligo-dT cellulose chromatography. The RNA was copied into double-stranded cDNA which was inserted in pBR322. Transformed cells were plated on nitrocellulose filters and replica filters were hybridised with labelled cDNA probes synthesised from poly A⁺ RNA of Ka and the virulent K geographical isolate (Kv). cDNA plasmids were selected representing RNAs of differing abundance. Increased levels of RNA molecule were found to correlate with avirulence.

However, the main thrust of this reference was to identify cDNAs corresponding to RNAs which hybridise in greater abundance with probes synthesised from poly A⁺ mRNA of the avirulent Ka strain. This reference did not disclose however preparation of polypeptides.

The present invention in one aspect resolves at least some of the problems associated with the prior art by providing a process which serologically determines a protective antigen and also raising a monoclonal antibody to that antigen. The process also includes cloning DNA sequences that code for polypeptides at least partially homologous with that antigen. The said process thereby provides DNA sequences, recombinant DNA molecules and methods for use of those sequences and molecules in the prediction of polypeptides displaying at least some of the immunogenic activity of that antigen.

By virtue of this invention, it is possible to obtain polypeptides displaying an immunological activity of the 12D3 antigen, for use in protective immunisation of animals, and more particularly cattle, against clinical Babesiosis. This recombinant DNA-produced polypeptide may also be used for the purpose of immunoassay and immunodiagnosis. This invention allows the production of novel polypeptides derived, modified or otherwise produced from the novel polypeptide in amounts and by methods hitherto not available.

As will be appreciated from the disclosure to follow, the DNA sequences and recombinant DNA molecules of the invention are capable of directing the expression of a polypeptide displaying at least some of the immunological activity of the native 12D3 antigen. Replication of these DNA sequences and recombinant DNA molecules in appropriate hosts also provides a means of amplifying DNA to yield quantities of DNA coding for the polypeptide in hitherto unobtainable quantities. The molecular structure of these genes may thus be readily determined. The polypeptide and corresponding DNA is useful, either as produced in the host or after appropriate derivatisation or modification, in composition and methods for detecting and improving the production of these products themselves and for use in immunoprotective, immunodiagnostic and immunoassay agents and methods.

The sequences of this invention are further characterised in that they permit the production of 12D3-like polypeptides in non-babesial hosts. In another aspect of the present invention there is provided antigen purified by immunoreactivity with the monoclonal antibody reactive with the above described monoclonal antibody.

The invention also includes within its scope an anti-idiotypic antibody displaying at least some of the immunogenic activity of 12D3 antigen.

In yet another aspect of the present invention there is provided DNA sequences coding for polypeptides having at least some of the immunoreactivity of 12D3 antigen wherein the sequences comprise cDNA sequences corresponding to babesial mRNAs having substantial homology with at least part of the Babesia gene or genes coding for antigen 12D3. Also within this aspect of the present invention are DNA sequences which hybridise to any of the foregoing DNA sequences, DNA sequences from whatever source including natural, synthetic or semi-synthetic sources, related by mutations including single or multi-base substitutions, deletions, insertions, inversions and 3′ or 5′ additions to any of the foregoing DNA sequences or inserts, and DNA sequences comprising sequences of codons which on expression code for a polypeptide displaying similar immunogenic properties to a polypeptide produced on expression of any of the foregoing DNA sequences.

In another aspect of the present invention are provided compositions comprising any of antigen 12D3 , the monoclonal antibody corresponding to antigen 12D3 and polypeptides expressed as above together with an appropriate vehicle such as an adjuvant, which compositions are variously useful as vaccines, immunoassay reagents and immunodiagnosis reagents.
The purified antigen may be used, on conjunction with a suitable adjuvant, as a vaccine, and suitable vaccination trials conducted.
In the present invention, antibody-purified antigen may be used to raise bovine antisera for serological analysis of the efficacy of the antigens produced fermentatively as is described hereinafter. The affinity purified antigen may also be used for immunodiagnosis of unknown sera.

Whilst affinity purified antigen may form an acceptable vaccine, in terms of industrial scale production this means of production is insufficient compared to fermentative processes using recombinant DNA technology. This inefficiency arises from the need to prepare large quantities of infected erythrocytes to source the crude antigen mixture.

Accordingly it is desirable to identify babesial genes coding for polypeptides having at least some of the immunogenicity of the antigen of interest such that the genes may be cloned and expressed in appropriate hosts.

There exists many ways by which the gene of interest may be identified. For example, the purified antigen may be partially amino acid sequenced, and corresponding DNA probes synthesised to screen a genomic or cDNA library to identify clones containing homologous DNA sequences.

The said DNA sequences through further manipulation may be expressed to give polypeptides exhibiting the desired immunological characteristics of the antigen.

Alternatively, the babesial gene may be identified by screening of a cDNA expression library constructed from the reverse transcription of babesial poly A⁺ mRNA using an antibody probe to identify clones expressing antigenic gene product. The library may then be used to provide inserts for expression vectors which are in turn used to transform appropriate hosts. The transformed host may be screened for other expression of the gene of interest.

In the process of the invention any suitable expression vector may be utilised as described hereinafter. Where the particular polypeptide of interest is most ably expressed by a eukaryotic host, it may be more appropriate to use the derivatised plasmids of a yeast such as Saccharomyces cerevisiae.

In the process of the invention any suitable host for the expression vector may be used. Examples of suitable hosts include yeast cells, mammalian cells, insect cells and bacterial cells. Transformants may be isolated by any appropriate means, for example, growth on selective media.

The transformed hosts may then be screened, either by detection of the desired expressed polypeptide using immunoassay or by probing either DNA or RNA probes.

In the preferred case where monoclonal antibodies have been raised against the antigen of choice, immunological screening of expressing recombinants is preferred.

The vector/host combination may be chosen such that the expressed polypeptide is just the translation product of the cDNA transcript of the poly A⁺ mRNA. Alternatively, the translation of the cDNA insert of the expression vector is placed under the control of a strong promoter for expression of a host protein by insertion of the c

DNA (in the correct reading frame and orientation) within the gene coding for that host protein. The result is that the host strongly expresses a fusion protein comprising the translation product of the cDNA insert coupled to at least part of the host protein. In this embodiment suitable use is made of a monoclonal antibody probe to screen a cDNA or genomic library.

The selected transformant may be grown up to yield sufficient quantities of expression product having the desired immunogenic properties for use in immunodiagnosis, immunoassay or vaccines.

It will also be appreciated in relation to this invention that one or more "reverse engineering" steps could be utilised in the inventive process wherein in some cases the antigen 12D3 or related antigens may be obtained from sera from vaccinated animals or infected animals using a Babesia expression library which may be cDNA or genomic in origin. In this case the sera would be used as a probe to screen the expression library to isolate 12D3 antigen or related antigens in a similar manner as described above.

In another aspect of the invention it will also be appreciated that the vaccine containing the antigen of interest could also be utilised as a starting material to eventually isolate the 12D3 antigen or related antigen. In this procedure the antigen from the vaccine could be sequenced to obtain a partial amino acid sequence and oligonuoleotide probes constructed from the amino acid sequence could be used to screen a Babesia and more particularly a B. bovis cDNA or genomic library to isolate a gene coding for a related antigen. By using recombinant technology one could then prepare the antigen in large amounts.

The invention also includes within its scope a test kit for diagnosis of babesiosis which includes the monoclonal antibody corresponding to antigen 12D3 or related monoclonal antibodies as well as selected reagents for detection of babesiosis antigen in body fluids such as blood or lymph. In this test kit any suitable label may be used to detect the antigen-antibody reaction and thus this may be IFA, RIA or more suitably enzyme immunoassay such as ELISA. A method of immunoassay using monoclonal antibody MAbA12D3 is also included within the scope of the invention.

It will also be appreciated that a monoclonal antibody similar or related to the monoclonal antibody MAbA12D3 (i.e. not necessarily reactive with the same epitope) may be obtained by using antigen 12D3 or a similar antigen or related polypeptide instead of the antigen obtained from a lysate of B. bovis infected erythrocytes as described herein.

A particularly preferred method for use in the present invention may include the following steps:
(i) purifying an antigenic fraction of Babesia;
(ii) sequencing the N-terminus of the purified antigenic fraction from step (i);
(iii) preparing one or more synthetic oligonucleotides based on the sequence obtained from step (ii);
(iv) preparing a cDNA library from m-RNA derived from Babesia;
(v) screening the cDNA library using the synthetic oligonucleotide prepared from step (iii) as a probe;
(vi) purifying positive clone(s) and subcloning the cDNA into an expression vector; and
(vii) expressing the clone(s) in the expression vector and obtaining the resultant antigen.

In step (i) suitably the antigenic fraction is purified in a similar manner to that described above in Australian Patent Specification 553779 wherein a monoclonal antibody is obtained from a lysate of Babesia-infected erythrocytes. Suitably the monoclonal antibody so obtained corresponds to an IgG species and more suitably an IgG1 species. In this manner clone A12D3 was isolated by limiting dilution of hybridoma cells.

After isolation of the monoclonal antibody it may be propagated in appropriate culture media such as tissue culture media (e.g. RPM1 1640) containing foetal calf serum stripped of bovine IgG harvested from the secreting cells by centrifugation. The supernatant may then be taken and antibody obtained therefrom was desirably concentrated using osmotic pressure differences (e.g. by being passed through a suitable ultrafiltration medium of 10,000 - 20,000 MW cut-off).

The antibody may then be attached to a ligand bound to an immobilised support. The ligand may be selected from Protein A, anti-mouse Ig, anion or cation exchange groups such as DEAE (diethlaminoethyl) or hydrophobic groups such as hydrocarbon or phenyl groups.

Suitable supports may be selected from Agarose, Sepharose, Sephacryl, Affigel, or nitrocellulose.

From the foregoing it will be appreciated that the antibody may be bound to ion exchange, hydrophobic or affinity ligands attached to the support which may be of any suitable type and which may be selected on the basis of which ligand is being utilised.

Suitably the antibody concentrate had a pH which was adjusted to a value of between 7.0-9.5 (preferably 8.6) before the antibody was attached to the ligand on the support. A preferred ligand is Protein A and a preferred support is Sepharose.

The antibody may be eluted in any suitable manner. An appropriate elutant may be selected from solutions of high ionic strength suitably having a pH greater than 7.0 or acidic solutions of low pH. An appropriate elutant may be HCl dissolved in glycine buffer, triethylamine, urea, diethylformamide, citrate/citric acid, citrate/phosphate and/or sodium thiocyanate.

It is also preferred to adjust the pH of the monoclonal antibody to the desired range of between 7.0 - 9.5 immediately after the purification procedure i.e. after the elution step or the other alternative purification procedures referred to above.

After the elution step the antibody may be purified by dialysis or other means such as freeze drying or ultrafiltration. The monoclonal antibody may then be analysed by appropriate means such as SDS ( sodium dodecylsulfate) mercaptoethanol electrophoresis, SDS-dithiothreitol electrophoresis or SDS-dithioerythritol electrophoresis. The aforementioned methods are suitably carried out under denaturing reducing conditions. An alternative analytic electrophoresis procedure may be carried out under denaturing conditions in the absence of a reducing or oxidising agent. If desired another suitable analytical procedure may be carried out under non-denaturing conditions utilising a suitable immunological technique such as the Western blotting test described hereinafter.

The purified antibody may then be attached to a suitable ligand bound to an immobilised support. Any of the ligands or supports discussed previously may be utilised in this step such as tresyl-activated Sepharose.

While SDS has been referred to above as a suitable detergent it will be appreciated that other detergents may be utilised in the electrophoresis such as lithium dodecyl sulfate.

While the preferred purification for the monoclonal antibody utilises a ligand bound to an immobilised support, it will be appreciated that other appropriate purification procedures may be adopted. For example a "salting out" procedure may be used with a precipitant selected from ammonium sulfate, polyethylene glycol or RIVANOL.

Alternatively the purification may be effected by suspension in solutions of low ionic strength e.g. water.

Yet again another purification procedure may utilise gel filtration to purify the monoclonal antibody on the basis of size differences. If ultrafiltration is used a suitable MW cut-off range is from 75,000 to 130,000 or greater than 200,000.

The purified monoclonal antibody may then be coupled to an appropriate ligand such as any one of those ligands previously described. Preferred ligand-support combinations are tresyl-activated Sepharose, cyanogen bromide activated-Sepharose, Affigel or Reactigel.

Alternatively the monoclonal antibody may be immobilised or insolubilised using cross linking agents such as glutaraldehyde or bis(sulfosuccinimidyl)suberate.

Alternatively the monoclonal antibody may be immobilised or insolubilised using biotinylated monoclonal antibody coupled to avidin-agarose or streptavidin-agarose.

The lysate of Babesia-infected erythrocytes may then be prepared in any suitable manner. Any one of the following procedures may be utilised:
(i) osmotic lysis wherein the cell membrane may be disrupted in solutions of weak ionic strength;
(ii) mechanical lysis e.g. sonic disintegration;
(iii) proliferation of parasites within the cell and existing therefore causing breakdown of the cell membrane; and
(iv) cell lysis wherein membranes may be lysed by specific lysins or cell antibodies.

Preferably in step (i) the cell lysate may be prepared as described by the methods referred to in Mahoney (1967) Exp. Parasitol. **20**, 232-41. Suitably, however, Babesia parasite infected cells may be subjected to differential lysis in hypotonic saline. Alternatively infected cells may be lysed in 5 volumes of distilled water by osmotic pressure using a freeze and thaw technique. Alternatively the infected cells may be lysed by the action of lytic agents such as saponin.

The lysate may then if desired be partially purified. This may be carried out in any suitable manner such as passing the lysate through insoluble media such as diatomaceous earth (Celite) or heparin-Sepharose This procedure removes contaminants and enriches the specific antigen.

The antigen then may be eluted from the insoluble media by appropriate elutants such as those described previously. However, a preferred method of elution is to increase the ionic stength with a suitable reagent such as sodium chloride in buffer.

The partially purified antigen extract may then be contacted with the monoclonal antibody which is suitably bound to a suitable support as described previously or may be present in cross linked form as described previously.

Subsequently the specific antigen which is now bound to the monoclonal antibody may be eluted therefrom by adoption of any of the elutants previously described. Suitably a washing agent that will not break the antigen-antibody bond is utilised prior to the elution procedure.The solution of specific antigen may have a pH of between 6.0-9.5 (preferably 7.4) before and after contact with the monoclonal antibody.

The specific antigen may then be analysed by any of the methods previously described for analysis of the monoclonal antibody. However preferably the specific antigen is analysed by two-directional electrophoresis using isoelectrofocusing in a first direction and SDS-type electrophoresis in a second direction as described previously.

The purified antigen obtained from the above procedure, preferably having a Mᵣ of 38,000 may have its N-terminal amino acid sequenced in any suitable manner such as by chemical or enzymatic cleavage of the antigen and subsequent separation by chromatographic methods, The sequence of each purified polypeptide may be determined by the Edman method. Preferably the analysis is carried out automatically in a sequenator. Preferably a synthetic oligonucleotide may be prepared having the following sequence:-
Methionine-Tyrosine-Aspartate-Glutamine-Asparagine-Glycine--Lysine as described hereinafter.

The cDNA library may be obtained from mRNA derived from Babesia in any suitable manner. Preferably cytoplasmic RNA is obtained from a suitable Babesia species such as B. bovis. In this procedure B. bovis infected erythrocytes are subjected to centrifugation and the resulting pellet treated with a suitable lytic agent such as guanidinium chloride. The resulting nucleic acids including DNA and RNA after further purification by centrifugation and use of caesium chloride reagent isolated the babesial RNA. Subsequently poly A⁺ RNA was prepared and CDNA prepared therefrom by reverse transcription.

Following preparation of Babesia cDNA this was then cloned into a suitable cloning vector which was preferably phage such a λgt10. If desired alternative cloning vectors could be used such as other phage or plasmids selected from pBR322, pUC, pGEM or pGEX. Suitably prior to cloning the CDNA is methylated, polylinkers such as EcoR1 incorporated therein and the CDNA subsequently digested by a suitable restriction endonuclease such as EcoR1. The resulting cDNA was then ligated to the cloning vector DNA by use of a ligase to form the desired cDNA library.

The oligonucleotide used to probe the cDNA library was suitably derived from the amino acid sequencing of the N-terminal portion of the antigen and was suitably labelled. Preferably a radioactive label was utilised such as ³²P.

Suitably since the cDNA was preferably amplified in a λgt10 library positive plaques were obtained after treatment with the oligonucleotide probe. In this regard positive clones detected by the aforementioned label were comprised of a hybrid of synthetic oligonucleotide and matching cDNA. subsequently the recombinant cDNA clones were obtained which were then subcloned into suitable expression vectors preferably selected from pGEM, pRIT, pUC, pUR, pGEM or pGEX, which were subsequently used to transform E. coli. When using pRIT as an expression vector the polypeptide may be prepared as a fusion protein coupled to protein A. When using pUC19 the desired polypeptide may be prepared as a fusion protein coupled to α-complementation Factor which is part of β-galactosidase. When using pGEX-3X, for example, the desired polypeptide may be prepared as a fusion protein coupled to glutathione-s-transferase.

Sequencing of bases of the positive clones were carried out in any suitable manner such as by the Sanger method or Maxam-Gilbert method.

### MATERIALS AND METHODS

### Chemicals

All chemicals were of analytical grade unless otherwise stated. Imidazole, either from Sigma or BDH, was recrystallised to a white powder from acetone. Dithiothreitol and benzamidine were purchased from Sigma Chemical Company. Electrophoresis chemicals and marker proteins were from Bio-Rad, LKB or BRL. "Milli-Q" reagent quality water (Millipore Corporations USA) was used throughout and all buffers were filtered through a 0.45m screen (Millipore) just prior to use.

### PREPARATION OF THE AFFINITY MATRIX.

Clone A12D3, an IgG1 subclass, was isolated by limiting dilution of hybridoma cells prepared from fusion of BalbC splenocytes with mouse NS-1 myeloma cells, by conventional techniques as previously described (3), with the exception that traces of bovine Ig were removed from foetal calf serum using protein A-sepharose (Pharmacia) Journal Immunological Methods **60**, (1983) 33-45.

The A12D3 clone was propagated in RPM1 1640 medium containing 10% foetal calf serum and the antibody was harvested from the secreting cells by centrifugation at 3000 x g for 10 mins. Antibody was purified from culture supernatant essentially by the method of Underwood et al (1983) J. Immunological Methods **60**, 33-45 1983. Approximately 9L of supernatant containing 10 mM azide, was concentrated to four lots of approximately 200 ml using an Amicon circulating concentrator fitted with a YM-10 membrane. The concentrate was adjusted to pH 8.6 with 0.5 M NaOH and allowed to recirculate overnight on a column of protein A sepharose (1.6 x 11.5 cm, flow rate 10 ml/h). The column was washed extensively with 50 mM Tris buffer pH 8.6. Bound protein was eluted with 500 mM glycine, pH 3.0 into tubes containing sufficient 1 M imidazole, pH 7.4 to neutralise each fraction. The column was re-equilibrated and the wash fraction extracted twice for antibody. The eluted fractions were pooled, concentrated by vacuum dialysis, and dialysed against 0.1 M NaHCO3 containing 0.5 M NaCl. The final product, 8 ml containing approximately 3 mg/ml protein (Bradford) was analysed by SDS/mercaptoethanol electrophoresis. The final product showed two major silver staining bands, at Mᵣ 55,000 and 25,0000. An equivalent sample was analysed by immunoblotting with affinity-purified, peroxidase-labelled goat anti-mouse IgG (H+L). Two major bands of Mᵣ 55,000 and 25,000 were evident.

The purified antibody was coupled to an equal volume of swollen tresyl activated-Sepharose 4B (Pharmacia) by essentially the manufacturer's instructions, except that the coupling reaction was followed by monitoring the removal of protein from the supernatant of a centrifuged aliquot. After an estimated 90% coupling (70 min) the reaction was stopped by blocking unreacted sites with 100 mM Tris, pH 8.0 and the gel further washed as per instructions. The antibody coupled gel was packed into a column (1.6 x 4.5 cm) and equilibrated in 50 mM imidazole, 150 mM NaC1, 10 mM sodium azide, pH 7.4. Just prior to use, the column was washed with the above buffer without azide followed by freshly prepared 50 mM triethylamine (Pierce, HPLC grade) pH 11.4, then re-equilibrated with starting buffer.

### PREPARATION OF CELITE

Diatomaceous earth for swimming pool filters ("Sentry" USA) was washed with water to remove fines and then washed with 5N HCl, and methanol. The slurry was packed into a column (5.0 x 27.0 cm) and equilibrated with running buffer (50 mM imidazole, 5 mM EDTA, 2 mM benzamidine, 1 mM dithiothreitol, pH 7.4)

### IMMUNOASSAY FOR 12D3

12D3 antigen was identified throughout the purification by spotting and drying 5 ml aliquots of each fraction on to nitrocellulose-backed millititre plates ("Millititer HA", Millipore Corporation USA) and performing a dot-blot assay as described by Hawkes et al Anal Biochem **119**, (1982) 142-147 with the exception that the blocking agent used was 5% (w/v) skim milk powder ("Carnation" Australia). Blank wells containing antigen but no primary antibody A12D3 were routinely run.

### 2D ELECTROPHORESIS.

2D electrophoresis was performed as described by O'Farrel J. Biol. Chem (1975) **250**, 4007-4021.

### DETAILS OF THE PURIFICATION.

All steps were carried out at 0-4°C unless otherwise stated.

B. bovis infected erythrocytes were harvested from splenectomised calves and concentrated as described previously (Mahoney, Experimental Parasitology **20**, 301-341 (1967)). The 100% infected intact red blood cells were diluted two fold with phosphate-buffered saline (PBS) and stored frozen at -70°C. The frozen cells (50 ml) were thawed under running tap water in the presence of an equal volume of 100 mM imidazole, 10 mM EDTA, 4 mM benzamidine and 2 mM dithiothreitol, pH 7.4 and sonically disrupted for 2 mins at 50 W in a cooled cell (Braun Labsonic, Model 1510). This treatment was sufficient to render the parasites non-infective (data not shown). The sonicate was centrifuged for 12 h at 100,000 x g and the supernatant applied to the celite column equilibrated with running buffer. The column was washed with the same buffer at 150 ml/h until protein was no longer detectable. The 12D3 antigen was then eluted with running buffer containing 1 M NaCl, total volume 300 ml, concentrated on an ultrafilter fitted with a YM 10 membrane (Amicon) to a volume of 50 ml, and dialysed overnight against 50 mM imidazole, 150 mM NaCl, 50% glycerol, pH 7.4.

After dialysis, the celite fraction was diluted with an equal volume of 50 mM imidazole, 150 mM NaCl, pH 7.4 (Affinity column running buffer) and the sample allowed to cycle at 50 ml/h through columns of Sepharose 4B (2.6 x 15 cm), Tris-blocked Tresyl-activated Sepharose 4B (1.6 x 2 cm) and A12D3-Sepharose 4B. The columns were washed with 200 ml of affinity column running buffer, the monoclonal antibody affinity column disconnected, and washed with 50 mM imidazole, 1 M NaCl, pH 7.4 (100 ml), followed by affinity column running buffer (200 ml).

Antigen was eluted from the column with 50 mM triethylamine-HCl pH 11.4 in 2 ml fractions containing 0.4 ml I M imidazole, pH 7.4. The eluate was concentrated to approximately 0.5 ml using Amicon "Centricon" concentrators with YM-10 membranes. The final material was frozen in liquid nitrogen and stored at -20°C.

### ELECTROPHORESIS

SDS electrophoresis was performed as by Tsang et al (1983) Methods in Enzymology **92** 277-391 except that samples were heated at 95 C for 20 minutes in the presence of 1% SDS and 1% mercaptoethanol. Immnunoblotting and silver staining procedures were performed according to Tsang et al (1983). Supernatants of monoclonal antibodies were used at 1:10 dilutions and goat anti-mouse antibody which was peroxidase labelled at 1:500 dilution.

### RESULTS

A purification scheme for a typical preparation is shown in Table I. Estimation of the protein is purely qualitative because of the small amounts of protein available for assay. However, it was observed consistently that dot blot assays using the crude extract showed weak activity which was markedly enhanced with fractions obtained after celite chromatography. Celite breakthrough and affinity column fractions were devoid of 12D3 activity, even after concentration and re-assay. Assuming no loss of yield throughout the purification, the purification represented an approximately 30,000 fold enrichment from the soluble 100,000 x g fraction. Final amounts of antigen were consistently 40-80 »g per preparation, approximately 2 »g per ml of 100% infected red cells.

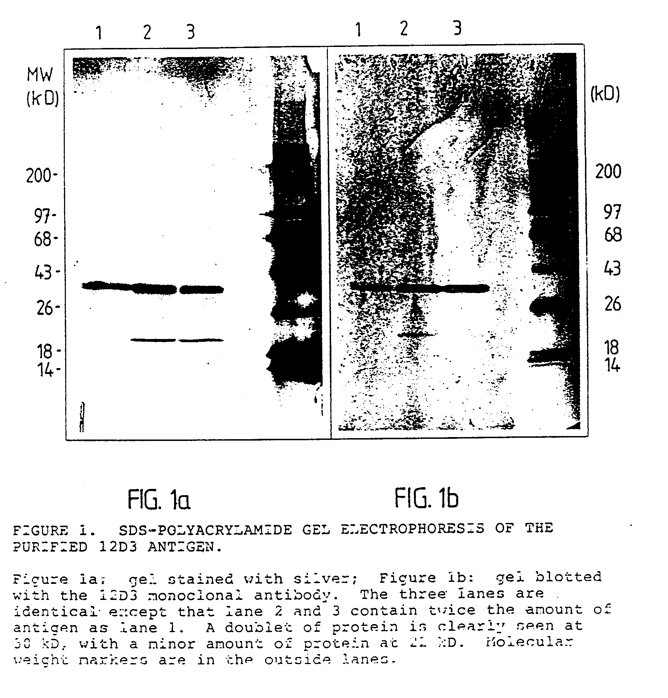

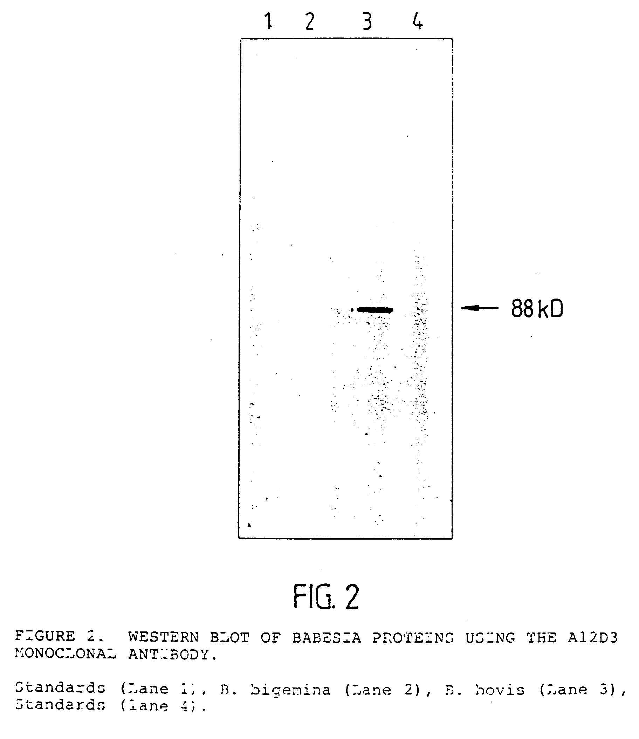

Figure 1 shows SDS-PAGE of the antigen under reducing conditions; there were consistently two dominant silver stained bands of approximately Mᵣ 38,000 which were just resolvable, and occasionally an additional minor band circa Mᵣ 22,000 (Figure 1a). A similar gel pattern was evident with Coomassie Blue R-250 staining. However with this dye the Mᵣ 22,000 band stained more intensely (Data not shown). Similar pattern was observed when blotting with the A12D3 monoclonal antibody was carried out, with the ca 22kD band blotting only very weakly. The Mᵣ 38,000 band consistently blotted with the A12D3 antibody. No other bands are apparent by either silver staining or immunoblotting. Data (not shown) demonstrated that the electroblotting profile as observed is A12D3 antibody dependent.

Crude 100,000 x g supernatant, and the fraction eluting with 1M NaCl from celite were electrophoresed and immunoblotted. It was seen that 12D3 is a single dominant antigen of approximately Mᵣ 38,000 and that the relative molecular weight does not vary throughout the purification. This suggests that no significant size modification of the Mᵣ subunit has occurred as a result of the purification.

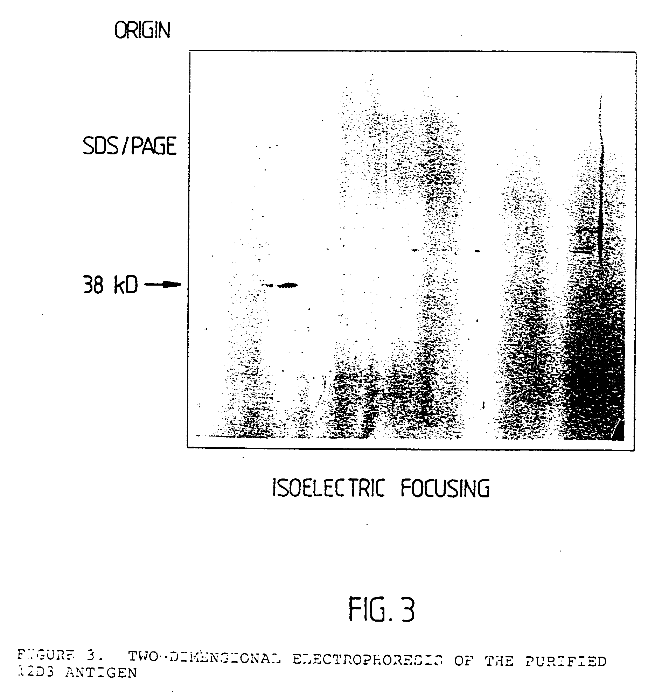

The affinity purified antigen was subjected to further analysis by 2D-electrophoresis (Figure 3). The silver stained gel showed one major spot of Mᵣ 38,000 and a number of minor spots, possibly antibody contaminants. In Figure 3 the gel was blotted with A12D3 antibody. As with conventional electrophoresis, only the protein visible by silver staining appeared to immunoblot. "Nonsense" monoclonals made to horse red blood cell antigens also had no reaction with the Mᵣ 38,000 spot.

The 12D3 antigen was injected with the adjuvant into cattle. After three months, a titre of 1:150,000 was obtained as judged by radioimmunassay, using established protocols (Wright et al, 1983) referred to above. Western blot analysis showed one major band Mᵣ 38,000 and a minor band Mᵣ 200,000 (data not shown). The high molecular weight band is consistently seen with "control" cattle experiencing an inflammatory reaction, and could be a consequence of the adjuvant (Dr B Goodger, Personal communication).

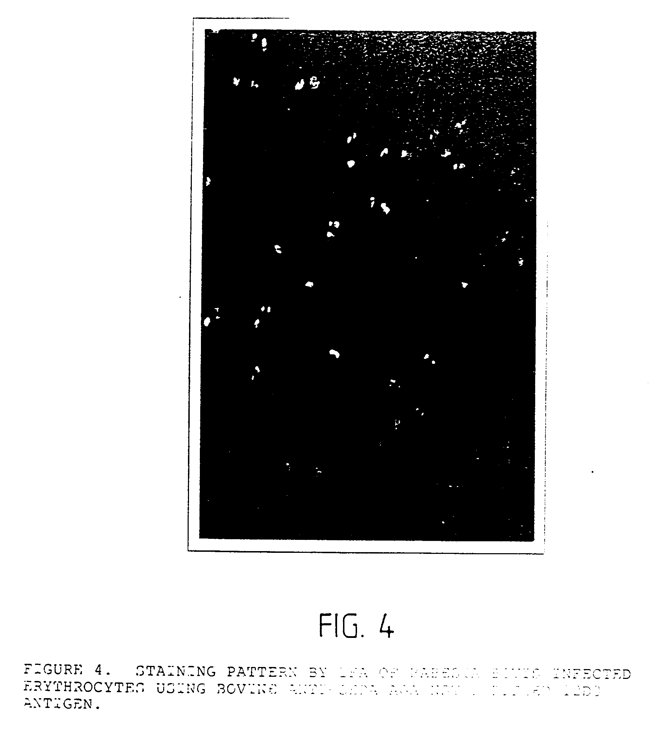

The IFA staining pattern is shown in Figure 4. Bovine antisera to the purified 12D3 antigen demonstrated predominantly parasite staining, consistent with the pattern as seen with the A12D3 monoclonal.

The purified 12D3 antigen was subjected to limited amino acid sequencing by the gas phase procedure on an Applied Biosystems Sequenator coupled to PTH amino acid detection by HPLC on a Dupont »Bondapak C₈ column. Sequence data for residues 3-25 is given in Table 2. For each cycle, two amino acids appeared in approximately equal amounts, and a minor contaminant at 10% of the total peak area was also discernible. Two polypeptide chains were apparent, in approximately the same amounts which ruled out an unequivocal assignment of each amino acids to a particular chain.

Having regard to the above disclosure the appearance of two polypeptide chains in approximately equal amounts in the final product ruled out an unequivocal assignment of each amino acid to a particular polypeptide chain. Further experimentation has allowed an unequivocal assignment of each amino acid to a particular chain, and the subsequent construction of relevant oligonucleotide probes. This further experimentation and the results obtained are the subject of the present addendum.

The material eluting from the affinity column constructed using the A12D3 monoclonal antibody was subjected to preparative SDS-mercaptoethanol polyacrylamide gel electrophoresis in the usual manner. At the end of the run, protein bands were visualised by immersion of the gel in ice-cold 0.25 M KCl for 16 h (Hager & Burgess, Anal Biochem **109**, 76 (1980) and the Mᵣ 38000 and Mᵣ 22000 proteins became evident as white bands against a black background. The respective bands were excised, and subjected to electroelution at 50V, 23 mA for 6 h in a custom-made electroelution apparatus. The basic details were similar to those described by Stearne et al J. Immunology **134**, 443-448 (1985). The electroeluted materials (0.8 ml) were transferred to siliconised borosilicate glass centrifuge tubes and 7.2 ml of -20°C methanol was added to each tube in two 3.6 ml aliquots. A precipitate developed and was allowed to stand over night at -20°C. The tubes were centrifuged at 10,000 x g for 1 h, the supernatants removed, and the precipitates dissolved in 200 ml 50 mM NH4HCO3 containing 0.05% SDS. A sample (20 ml) was removed for analytical SDS-mercaptoethanol polyacrylamide gel electrophoresis and the rest stored at -20°C. Analytical SDS-mercaptoethanol polyacrylamide electrophoresis resolved subunits Mᵣ 38000 and Mᵣ 22000 respectively with no apparent contamination. The purified Mᵣ 38000 protein was subjected to limited amino acid sequencing by the gas phase procedure on an Applied Biosystems Sequenator as previously described.

Sequence data for residues 1-29 for the Mᵣ 38000 protein are given in Table 3.

The unequivocal assignment of amino acids to the Mᵣ 38000 polypeptide chain has enabled the construction of a nucleotide probe for gene bank screening. That chosen is shown in the "Preparation of the Probe". It will also be appreciated that the babesiosis component of the invention includes within its scope not only a protein or peptide including the abovementioned amino acid sequence but also artificially created derivatives (e.g. by peptide synthesis or by recombinant DNA procedures including nucleotide substitution, insertion or deletion).

It will also be appreciated that other species of Babesia may be utilised other than B. bovis. For example, B. canis, B. ovis, B. divergens, B. equi or B. bigemina may be utilised. Also it will be clear to the man skilled in the art that particular strains (e.g. Samford or Lismore strain) of a particular Babesia species (e.g. B. bovis) may be utilised. This will include for example naturally or artificially induced mutants of the aforementioned strains or species.

It will also be appreciated that the invention also includes within its scope any antigens recognised by the A12D3 monoclonal antibody which are also characterised by the data set forth below. The invention includes within its scope the monoclonal antibody per se.

### VACCINATION TRIAL WITH A12D3 NATIVE ANTIGEN

Fifteen castrated adult Bos taurus male animals were purchased from an area known to be free from Boophilus microplus, the tick vector for B. bovis and B. bigemina in Australia. The sera of the animals were then screened by both ELISA and IFA to determine that their sera contained no antibodies to either Babesia species. The susceptible animals were then divided into three groups, each of five cattle.

Group 1 received 1 »g of affinity purified native 12D3 antigen in 1 ml of normal saline and 1 ml of freunds Complete Adjuvant (FCA) as a water-in-oil emulsion by subcutaneous injection on day 0 and 28.

Group 2 received 10 »g of the antigen in 1 ml of normal saline and 1 ml FCA as a water in oil emulsion on days 0 and 28.

Group 3, the control group received 1 ml normal saline and 1 ml FCA as a water-in-oil emulsion on days 0 and 28.

Serum samples were taken from all animals on days 0,14,28,42,49,and 56 and tested by both ELISA and IFA for antibodies. Animals were challenged with 1 x 10⁸ virulent B. bovis, of the Lismore strain (heterologous). The mean IFA titres day 56 were
Group 1 1/200
Group 2 1/400
Group 3 <1/50
All animals vaccinated with the 12D3 antigen had specific parasite staining with IFA, a similar staining pattern to that seen with the monoclonal antibody A12D3. ELISA titres were weakly positive with all vaccinated animals.

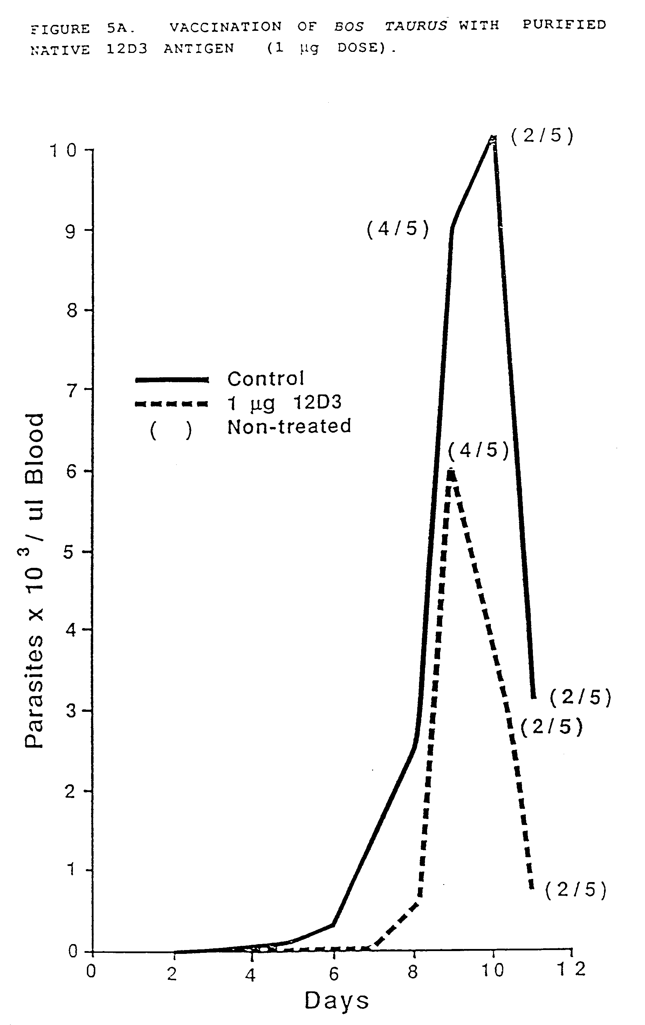

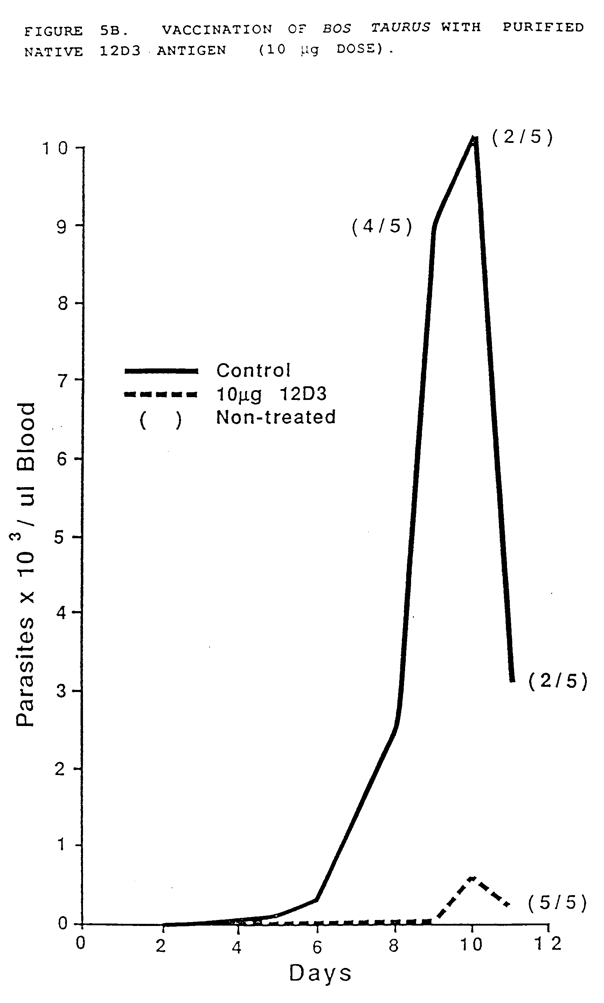

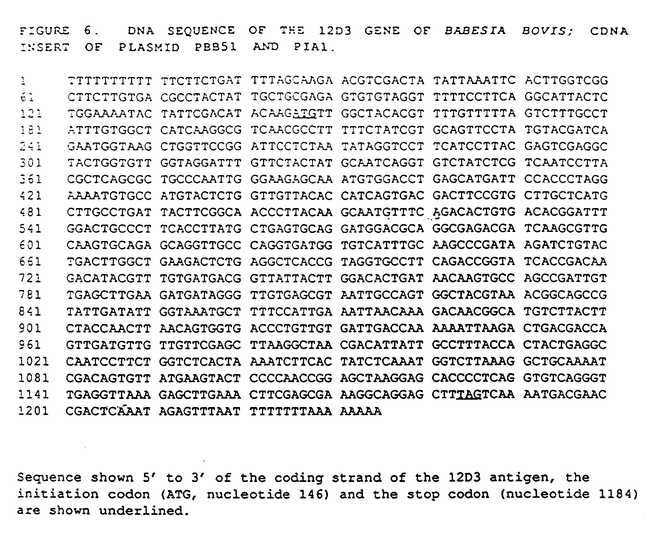

Upon challenge there was a two-day delay in the onset of parasitaemia in groups 1 and 2 which was highly significant statistically (p<0.01). However, by day 6 post-infection all animals had detectable parasitaemias. Parasitaemias of group 1 animals were not significantly different from those of the controls (group 3) by day 9, and 3/5 animals in both group 1 and group 3 were treated on days 10 and 11 (Figure 5a). The parasitaemias in group 2 (Figure 5b), however, were significantly lower than those in the control group for the duration of the experiment, the mean maximum on day 9 being 10,167 parasites/»l of blood in group 3 and 578 parasites/»l blood in group 2. No animals in group 2 required treatment, whereas 3 of 5 controls were treated with a babesiacide. These results are significant in that they show a dose response effect with the native 12D3 antigen and as little as 1 »g induced a significant delay in the onset of parasitaemia. Moreover, sera from these animals immunoblotted both native and recombinant 12D3 antigen.

### ISOLATION OF TOTAL RNA FROM BABESIA BOVIS-INFECTED ERYTHROCYTES

Blood was obtained from a calf hyperinfected (10-15%) with the Samford strain of Babesia bovis. The whole blood was spun down to pellet the erythrocytes. The pelleted erythrocytes were resuspended in five volumes of PBS pH 7.2 by inversion and the centrifugation repeated to effect washing of the erythrocytes. This step was repeated a further time to complete washing of the erythrocytes. The erythrocytes were then pelleted as before.

To prepare 100% infected cells, samples of the pelleted erythrocytes were treated with five volumes each of NaCl solutions which varied in concentration from 0.15 to 0.85% NaCl to determine the concentration of saline effecting lysis of uninfected cells preferentially to infected cells. In this case the concentration determined was 0.35% NaCl.

The remaining erythrocytic pellet was treated with five volumes of 0.35% NaCl for 15 minutes and then centrifuged (GSA rotor, Sorvall, 3000 rpm, 20 minutes, room temperature) to pellet the whole infected cells. The supernatant containing "ghosts" of uninfected cells and cell debris was discarded.

The pellet was resuspended in one volume of PBS pH 7.2 and the resulting suspension passed through a Whatman CF11 cellulose column, pre-equilibrated with PBS, to remove the white cells and platelets. The erythrocytic band of the column is highly visible and generally elutes with two to three column volumes of PBS.

The 100% infected erythrocytes were pelleted by centrifugation at room temperature in a Sorvall GS4 rotor (3000 rpm, 15 minutes) and the supernatant was discarded. The pellet was resuspended to 50% (v/v) of infected cells in PBS.

10 ml of infected cell suspension were placed in a polypropylene centrifuge to which was added 10 ml of guanidinium isothiocyanate stock (GI mix) at 60°C. The tube was maintained at 60°C in a water bath with gentle mixing until the solution went very viscous, indicating that total lysis of the erythrocytes and parasites had taken place with concomitant release of babesial DNA. The tube was removed from the bath and sonicated (100 Watts, 1 minute) to reduce the viscosity by shearing the liberated DNA.

25 ml of phenol (equilibrated with 1 M Tris/HCl, pH 7.4) was added to the tube and incubated at 60°C with thorough mixing. 12.5 ml of buffer (0.1 M sodium acetate, pH 5.2; 10 mM Tris/HCl, pH 7.4; 1 mM EDTA, pH 8.0) at 60°C was added to the tube with thorough mixing. 25 ml of chloroform/isoamyl alcohol (24:1) were added to the tube and the tube was shaken vigorously for 10-15 minutes at 60°C.

The tube was cooled on ice for 45 minutes and then centrifuged (GSA rotor, Sorvall, 5500 rpm, 15 minutes, 4°C) which resulted in the separation of two phases. The aqueous (top) phase was recovered, avoiding the debris at the interface, and was re-extracted with an equal volume of phenol/chloroform/isoamyl alcohol (25:24:1) at room temperature for 5 minutes. The mixture was centrifuged (SS34 rotor, Sorvall, 10,000 rpm, 10 minutes, room temperature) and the aqueous phase was again recovered. The aqueous phase was further extracted with chloroform/isoamyl alcohol (24:1) at room temperature. The mixture was centrifuged for 1 minute at 3000 rpm at room temperature in an MSE bench top centrifuge and the aqueous phase recovered.

Two volumes of absolute ethanol at -20°C were added to the aqueous phase and incubated overnight at -20°C to precipitate the nucleic acids. The nucleic acids were recovered by centrifugation (SS34 rotor, Sorvall, 10,000 rpm, 20 minutes, 4°C).

The recovered pellet was redissolved in 12 ml of double-distilled water and 4.8 grams of caesium chloride (Nucleic Acid Grade, Boehringer Mannheim) were added and dissolved. The resulting mixture was centrifuged (SS34 rotor, Sorvall, 10,000 rpm, 10 minutes, room temperature) and the supernatant was recovered.

To a 12 ml polypropylene centrifuge was added 4 ml of 5.7 M caesium chloride. The clarified nucleic acid solution was carefully layered on top of the caesium chloride solution and the tube was centrifuged (TST 41.14 rotor, Kontron, 30,000 rpm, 16 hours, 17°C). The supernatant was removed by careful aspiration and decantation with a pasteur pipette leaving an opalescent RNA pellet.

The RNA pellet was redissolved in 400 »l of sterile, double distilled water and precipitated by the addition of 40 »l of 3 M sodium acetate, pH 5.2 and 880 »l of absolute ethanol, and storage at -20°C overnight.

### CHARACTERISATION OF BABESIA TOTAL RNA.

The RNA was recovered by centrifugation (Eppendorf, 15 minutes, 14,000 rpm, 4°C) and the RNA pellet was redissolved in sterile, double distilled water. The ratio of the Absorbance at 260 nms to the Absorbance at 280 nms was taken as a measure of the purity of the RNA and was shown to be 2.0.

Analysis of the RNA by electrophoresis on an agarose gel indicated sharp 18S and 28S ribosomal bands and RNA of high molecular weight.

### PREPARATION OF POLY A+ RNA.

PolyA⁺ RNA was prepared using oligo-dT cellulose according to the procedures of Aviv, H. and Leder, P.(1972) Proc. Natl. Acad. Sci **69**, 1408-1412. as detailed in Molecular Cloning: A Laboratory Manual, Maniatis, T., Fritsch, E.F. and Sambrook, J.(1982) Cold Spring Harbor Laboratory. The preparation of RNA from Babesia bovis has also been described in a previous patent:

### SYNTHESIS OF cDNA FROM POLY A⁺ RNA.

The synthesis was conducted using a kit (Amersham International plc), which was based on the procedures of Gubler, U. and Hoffman, B.J., (1983) Gene **25**, 263-269.

The instructions for the synthesis kit were followed exactly. The components were stored at -20°C until required and thawed at room temperature and then stored on ice until use. The polyA⁺ RNA (1 »g) in distilled water was heated to 70°C and then cooled rapidly on ice before synthesis of the cDNA took place. At the completion of the the cDNA synthesis the cDNA reaction mixture was loaded onto a column of Sephadex G-50 (Pharmacia, Nick column) previously equilibrated with STE buffer (TE containing 10 mM NaCl) and eluted with STE buffer. Fractions of 400 »l were collected and and a scintillation counter used to determine the amount of radiation by Cerenkov counting. A sample of the extracted aqueous phase was kept and similarly counted.

The cDNA was eluted in the second fraction and the unincorporated nucleotides were eluted in fraction 4-5.

Measurement of the incorporation of labelled dCTP (2%) indicated that 560 ng of double-stranded cDNA was obtained indicating a 28% yield of cDNA based on the amount of mRNA .

### CLONING OF cDNA WITH λgt10.

cDNA was prepared and cloned into λgt10 using the Amersham⁻ λgt10 cloning kit according to the following procedures.

The babesia cDNA (1 »g) was methylated using EcoR1 methylase in the presence of S-adenosylmethionine for 60 min at 37°C, followed by inactivation of the methylase at 70°C for 10 min. EcoR1 linkers were attached to the cDNA by ligation overnight with T4 DNA ligase, followed by inactivation at 70°C for 10 min.

The linkered cDNA was digested with EcoR1 (37°C, 5 hr) and the excess linkers removed by gel chromatography. Fractions containing the cDNA as judged by Cerenkov radiation were ethanol precipitated. The precipitated cDNA (400 ng) was ligated into λgt10 arms in the presence of T4 DNA ligase (10 units) at 15°C overnight.

The ligation mix was packaged using the in vitro packaging mixes supplied by Amersham for 2 hrs at room temperature, at which stage 500 »l of sterile media were added.

The number of recombinants was determined by titration on two strains: L87, which allows both non-recombinants and recombinants to grow and NM514 which allows only recombinants to grow. An analysis of the data indicated that the library consisted of 363000 plaques of which 94% were recombinants.

Twelve plaques were selected and used to infect L87 cells until lysis occurred. The lysed cells were centrifuged, 5000 rpm, 10 minutes to remove cell debris and phage DNA was prepared from the supernatant. The DNA was digested with EcoR1 enzyme and electrophoresed through 0.8% agarose in the presence of ethidium bromide(0.5 »g/ml). Visualisation of the gel by exposure to UV light indicated that at least 8 of the 12 clones contained inserts ranging from 600 - 1000 bp.

### SCREENING OF THE LIBRARY.

### Preparation of probe.

A synthetic oligonucleotide was designed based on an amino acid sequence obtained from the N-terminal portion of a purified sample of 12D3 protein obtained from Babesia bovis-infected red blood cells (see sequence of 12D3 protein). The sequence selected for the preparation of the oligonucleotide was;
-Methionine-Tyrosine-Aspartate-Glutamine-Asparagine-Glycine--Lysine- because it required the least degenerate (i.e. the least number of DNA sequences to be prepared allowing for multiple codons). The DNA sequence prepared was complementary to the coding strand and is (reading from the three prime end):
3′-TAC-AT(A or G)-CT(A or G)-GT(T or C)-TT(A or G)-CC(A,T,G or C)-TT

### Labelling of the synthetic oligonucleotide

The synthetic oligonucleotide was labelled in the following manner:
20 pmoles of the oligonucleotide was prepared in 23 »ls of water, to which was added 5 »l of ten times kinase buffer, 20 »l of τ³²P-ATP (3000 Ci/mmol), and 1 »l of T4 DNA polynucleotide kinase (10 units). The reaction mix was incubated for 1 hour at 37°C. The reaction was stopped by the addition of EDTA and chromatographed on a Sephadex column to remove unincorporated ³²P.

### Screening of library

45,000 recombinants of the library in λgt10 were plated out on three large petri dishes containing LB agar using NM514 plating cells. Plaques were allowed to grow at 37°C for 7 hours at which time the plates were removed from the incubator and placed in the refrigerator. Copies of the plaques were made by transferring to discs of Hybond N (Amersham) in duplicate and marking the plates and the filters with India ink and a needle. The filters were denatured with 0.5 M NaOH, 1.5 M NaCl; followed by neutralisation with 0.5 M Tris, 1.5 M NaCl and rinsing in 2XSSC. The filters were air dried briefly and exposed, DNA side down, to a transilluminator (256 nms) for 3.5 minutes.

The filters were then prehybridised in the following buffer system;
6XSSC, 5xDenhardts solution, 0.5% SDS 20»g/ml denatured salmon sperm DNA at 43°C for four hours. The filters were then hybridised overnight with labelled synthetic oligonucleotide (specific activity 30 million dpm/pmol) at a concentration of 2 million dpm/ml of hybridisation fluid (same recipe as prehybridisation fluid). The filters were then washed with 4XSSC containing 0.1% SDS, three times for 40 minutes at 43°C. The filters were then exposed to X-ray film (Kodak X-AR) at -80°C overnight using two intensifying screens.

Positive plaques were indicated if the signal, was present on each of the duplicate filters. These plaques were picked using a sterile pasteur pipette and resuspended in 1 ml of sterile medium containing a drop of chloroform. Seven positives were so identified and replated using NM514 cells and the cycle of prehybridisation, hybridisation and washing repeated as above. Six of the seven clones were successfully hybridised in this second screen and positive plaques were picked as described above. In this way the recombinant Babesial cDNA was purified. For each of the plaque purified clones, a single plaque was picked and resuspended in 1 ml of sterile medium, 200 »ls of the resuspended plaque was plated out on a large plate of LB medium using NM514 cells and grown until confluent lysis had been obtained. The plate was then overlayed with 8 mls of cold sterile medium and stored in the refrigerator overnight to allow the phage to diffuse into the medium. The medium was recovered with a sterile pipette and centrifuged for 10 min at 10,000 rpm at 4°C. To the clarified supernatant was added an equal volume of polyethylene glycol (20% v/v) and 2M NaCl, and then mixed thoroughly. The mixture was allowed to sit on ice for 60 minutes, and then centrifuged for 20 min at 10,000 rpm at 4°C to pellet the phage. The supernatant was decanted off and the tube allowed to drain thoroughly before resuspending the pellet with 800 »ls of sterile medium. A centrifuge tube was prepared containing a 1 ml cushion of 5 M CsCl in sterile medium upon which was layered a 3 ml shelf of 3 M CsCl in sterile medium. The resuspended phage was layered onto the top-most shelf and the tube was centrifuged in the TST 55.5 Kontron rotor for 1 hour at 30,000 rpm at 20°C.

The phage was seen to band at the interface between the 3 M CsCl shelf and the 5 M CsCl cushion, and with the aid of a collimated light source the phage band was recovered using a 1 ml syringe fixed with a 21 gauge needle injected through the tube beneath the phage band.

DNA was prepared from the phage according to the formamide method of Davis, Botstein and Roth (A Manual for Genetic Engineering: Advanced Bacterial Genetics, Cold Spring Harbor, 1980) The DNA was analysed by digesting 5 »ls of the dissolved DNA with EcoR1 restriotion enzyme and electrophoresing through an agarose gel with DNA size markers as a control. Each of the samples contained inserts clone 1 (1.15 kbp), clone 2 (0.47 kbp), clone 3 (0.93 kbp) clone 4 (2.8 kbp, 1.85 kbp, 0.8 bp) and clone 5 (1.06 kbp).

The DNA insert was subcloned into the vector pGEM 4, or pGEM 3Z or pGEM7Zf (Promega Biotec) and transformed into cells JM105 or JM83 made competent using calcium chloride. Positive clones were selected on media containing ampicillin, X-gal and IPTG by the presence of a white colour. Clones were toothpicked into LB medium containing ampicillin and grown overnight with shaking at 37°C. DNA was prepared from these cultures using the rapid DNA method of Birnboim and Doly as described in Maniatis, Fritsch and Sambrook; (Molecular Cloning: A Laboratory Manual, Cold Spring Harbor, 1982). Plasmid DNA prepared in this manner was named pBb11, pBb21, pBb31 and pBb51 for clones 1,2,3 and 5 respectively referred to above. The DNA was prepared for sequencing by denaturing with NaOH, neutralising and ethanol precipitating. The pellet was recovered by centrifugation and redissolved in 6 ml of distilled water, 3 »l of sequencing primer(10 mg/ml) corresponding to either the T7 RNA polymerase promoter sequence or the SP6 RNA polymerase promoter sequence, and 1 »l of 10 X Klenow buffer(100 mM Tris HCl, pH 7.5, 500 mM NaCl). The dissolved DNA was equilibrated at 37°C for at least an hours The DNA was sequenced using the dideoxy method of Sanger and using the reagents and procedures of Promega Biotec.

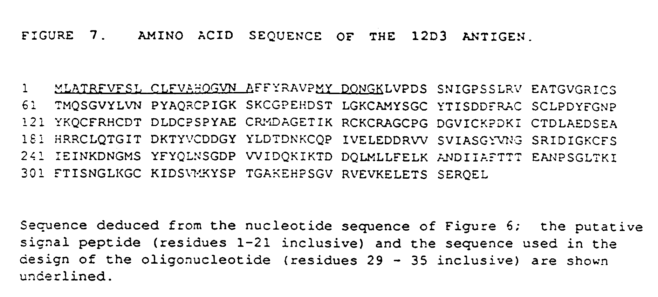

The entire DNA sequence of clone 5 (called pBb51, Figure 6) was determined by preparing deletions of the plasmid using exonuclease III and mung bean nuclease according to the procedures of Stratagene. Sequence of the terminal regions of the clone 3 (pBb31) and clone 4 (pBb41) were also determined to verify their identity. All three clones contained identical sequences and presumably were transcribed from the same gene.

When the DNA sequence was conceptually translated (Figure 7), an open reading frame was detected that corresponded to the amino acid sequence determined on the purified antigen. This confirmed that this cDNA coded for the 12D3 antigens An analysis of the amino acid sequence indicated a potential signal peptide at the N-terminal region of the antigen which may be involved in the secretion of the antigen. This sequence of protein contained all the features usually found with a signal peptide including a charged residue in the first five amino acids, a long stretch of hydrophobic residues, followed by a helix breaker (glycine, or glutamine) and an alanine at the site of cleavage. The site of cleavage is confirmed by a comparison of the sequence obtained from the protein and the sequence deduced from the cDNA. In addition, there is one potential site for N-glycosylation.

An important characteristic of the 12D3 antigen is that it contains a large number of cysteine residues in the mature protein (21 total)(there is also one cysteine in the signal peptide). These residues are clustered in the N-terminal half of the protein and the spacing of some of them has a remarkable similarity to the EGF (epidermal growth factor) domains of a variety of proteins. Two such EGF-like domains may be identified in the 12D3 antigen. Given that there is an odd number of cysteine residues in the mature protein, then there must be at least one not involved in disulfide bond formation. Aside from this structural similarity of a few of the cysteine residues, there is no other significant similarity of the 12D3 antigen with any known antigen or protein in the data-bases.

### EXPRESSION OF 12D3 ANTIGEN IN ESCHERICHIA COLI

The expression of 12D3 antigen was tested in a number of vectors of Escherichia coli. Three different expression vectors have been examined to demonstrate the generality of the principle and recombinant antigen from two of these has been tested in vaccination trials using beef cattle (Bos taurus). Expression of the antigen in E. coli was obtained in the following manner.

### Expression with pUC9

The insert cDNA of pBb31 contains the coding region for all but the first several amino aids of the signal peptide. The EcoR1 site allows the cDNA insert to be in frame with the genes of number of expression vectors. Thus, the cDNA was cloned into pUC9 (EcoR1 site) and expression of the fusion protein (with the α-complementation factor) achieved with induction by IPTG was achieved. Only small yields of fusion protein were found in the supernatant after lysis with lysozyme, and it was subsequently discovered that most of the fusion protein could only be solubilised using SDS. Fusion protein produced after treatment of cells with lysozyme blotted specifically with the A12D3 monoclonal antibody. For the purposes of a vaccination trial, flasks of LB broth containing ampicillin (20 »g/ml) and IPTG (25 »g/ml) were inoculated with 1/100 volume of a saturated culture of pUC19D3 in JM83. The cultures were grown overnight with vigorous shaking and the bacteria recovered by centrifugation. The pellet from a total of 27 litres of culture was resuspended in lysis buffer (25 mM Tris, 50 mM glucose, 10 mM EDTA, pH 8.0) and 2 mg/ml lysozyme. This suspension was then brought to 37°C for 5 minutes and was immediately pooled on ice and centrifuged to remove the bacterial debris. Ammonium sulfate was added to the supernatant and the precipitate was removed by centrifugation. Ammonium sulfate was added to give 50 g/ml and this preoipitate was recovered by centrifugation and redissolved in 50 mls of phosphate buffered saline.

### Expression with pRIT5

The same cDNA was inserted into the vector pRIT5 (Pharmacia) and expression achieved as a fusion with Protein A. The recombinant protein in this case also blotted with the monoclonal antibody A12D3, but low level expression of soluble protein only was detected.

### Expression using pGEX-3X

Similarly the cDNA was inserted into the vector pGEX-3X so that that 12D3 antigen was expressed as a fusion protein with glutathione-s-transferase. Expression in the vector, pGEX-3X gave only low level of expression of soluble protein whereas extraction with SDS indicated that upwards of 10% of total protein might be 12D3 fusion protein. This compared with the pUC19D3 fusion protein above. It was evident that the majority of the protein was expressed as a high molecular weight aggregate associated with membranes. Methods for the solubilisation of the 12D3 fusion protein were examined. It was discovered that the fusion protein could not be solubilised by sonication alone, or by sonication in the presence of Triton X-100. The 12D3 fusion protein was solubilised using sonication in the presence of 0.5% SDS and 0.1% β-mercaptoethanol. For the purpose of a vaccination trial, the sonicate was clarified by centrifugation, and the solubilised material was concentrated using ammonium sulfate (10% w/v). The precipitate was redissolved in PBS and then dialysed against several changes of PBS at 4°C. 50 mls of 10% pure 12D3 fusion protein at a protein concentration of 2.5 mg/ml was obtained from 3 litres of culture. The 12D3 fusion protein appeared to be soluble at this stage.

### THE 12D3 ANTIGEN IN STRAINS OF BABESIA BOVIS AND SPECIES OF BABESIA

### Strains of Babesia bovis

The presence of a gene encoding for the 12D3 antigen in a number of strains of B bovis was demonstrated in the following manner. Samples of DNA were obtained from the following strains (Samford, Lismore, Ka) and digested with the enzyme EcoR1 and electrophoresed through an agarose gel. The gel was denatured with alkali and neutralised and the DNA transferred to Hybond N nylon membrane. The filter was air-dried and the DNA baked onto the filter at 100°C.

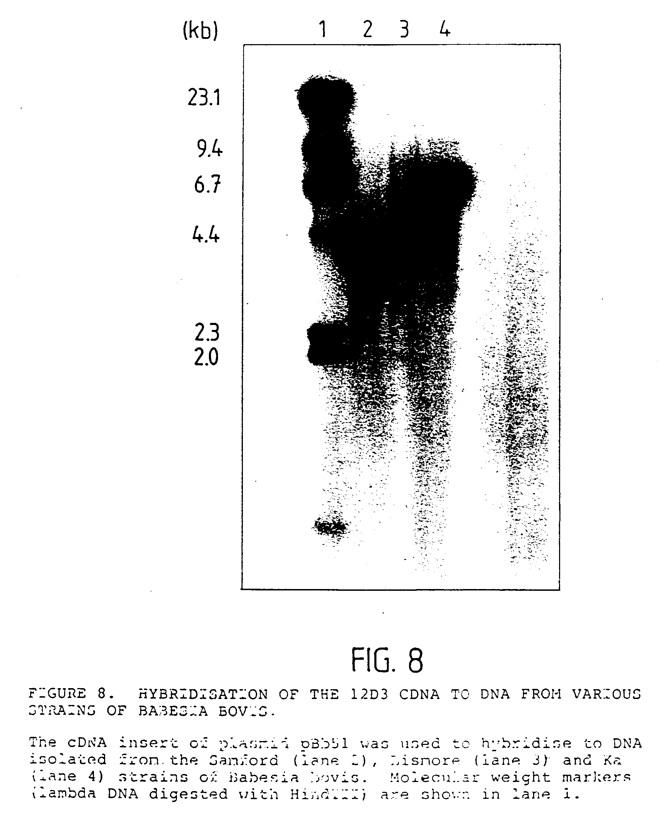

The filter was prehybridised in the following buffer (50% formamide, 5XSSPE, 5XDenhardts, 0.5% SDS, 20 »g/ml salmon sperm DNA at 42 C) and hybridised in the same buffer but with the inclusion of labelled cDNA obtained from pBb51. The filter was washed at 42°C with 0.1XSSC,0.1% SDS. An examination of the X-ray film (Figure 8) indicated a single band of hybridisation of size 3.6 kbps in the Samford and Lismore strains, and a single band of size 4.8 kbps in the Ka strain. Since the hybridisation and washings were carried out at high stringency the probe must be hybridising to a DNA sequence which is quite similar to the the 12D3 gene. Since the cDNA was obtained from the Samford strain it is likely that the remaining strains also contain a gene which encodes for a Samford-like 12D3 antigen. Significant protection was obtained in the vaccination trials using the Lismore strain to ohallenge a Samford antigen and this is consistent with the 12D3 antigen being present in all strains of B. bovis.

### Species of Babesia (Babesia bigemina)

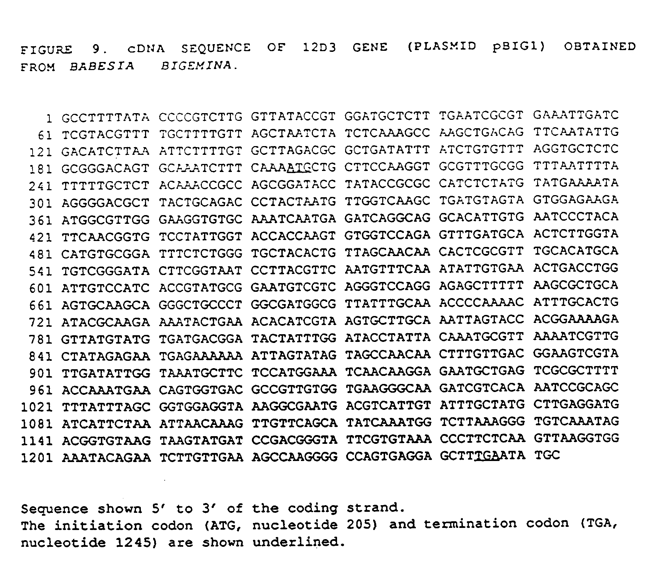

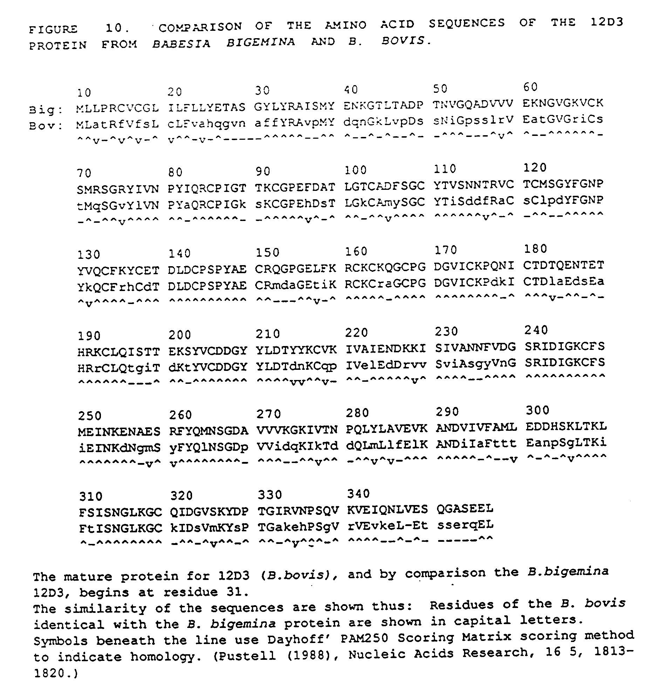

A cDNA library was prepared from mRNA of B. bigemina using the same procedures as described in this document for B. bovis, except that hexanucleotides were used to randomly prime the first strand DNA synthesis on B. bigemina mRNA. The library was screened using the cDNA obtained from pBb51 which encodes the 12D3 antigen, using conditions of hybridisation and washing such that 60% similarity between DNA duplexes would be required. A number of clones was obtained from the library using these procedures and some of these were suboloned into pGEM7Zf for sequencing as described for the B. bovis antigen. The DNA sequence, shown if Figure 9, from one of these clones (designated pBIG1) indicated high similarity with the 12D3 gene of B. bovis, and when the sequence was conceptually translated it yielded an amino acid sequence that maintained a high degree of homology. A comparison of the sequence of the B. bovis and the B. bigemina 12D3 proteins is shown in Figure 10. It was noted that the spacing and occurrence of the cysteine residues of the putative EGF-like domains was identical between the antigens, with a high degree of homology in parts of this region (70-80%). It can be predicted with reasonable certainty that those regions where the amino acid sequence is conserved between the two strains indicate domains which are important to the function of the protein.

It is evident that the cDNA from 12D3 (pIA1, pBb51) could be used to obtain similar genes form a variety of Babesia species, including B. ovis, B. canis, B. divergens and B. equi. It is also likely that the protein encoded by these genes could be used equally as well as vaccine candidates against these and other species of Babesia.

### Immunochemistry

The A12D3 monoclonal antibody had been used in the IFA to demonstrate cross-reactivity with other species. Staining patterns (predominantly whole parasite only) similar to those described earlier in this patent for B. bovis were demonstrated in B. bigemina, B. canis, B. equi and B. ovis. Polyvalent sera raised in cattle immunised with pure native 12D3 antigen (as described earlier) were also used in IFA and again cross-reactions were seen with all of the above mentioned species.

ELISA was also performed on B. ovis antigen using A12D3 monoclonal. This reacted, albeit weakly, at a similar level to that seen in a similar crude B. bovis antigen.

These data, taken together with the DNA data above are strongly supportive of the view that this antigen is highly conserved across all Babesia species. It can be predicted with reasonable accuracy that the 12D3 antigen as isolated from each species or produced through other means such as genetic engineering would be a suitable component for a vaccine against the respective Babesia organisms.

### TESTING OF 12D3 AS A VACCINE

Material from the pUC9 and pGEX-3X expression systems was examined in vaccination trials by vaccination with the recombinant protein followed by challenge with virulent Babesia. In each case, significant protection was achieved as measured by the difference in parasitaemia between the control animals (vaccinated with adjuvant) and those vaccinated with recombinant antigen. The degree of protection was not as significant as when using purified native antigen.

### pUC19D3

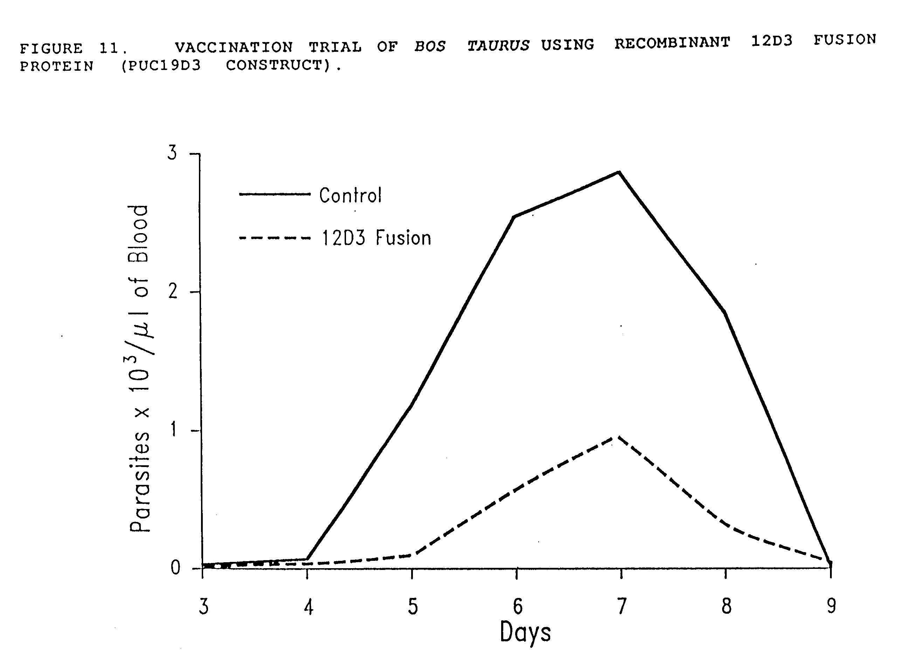

Two groups each of five adult castrated Bos taurus animals purchased from a tick free area and shown by both ELISA and IFA to not have antibodies to B. bovis were vaccinated twice, four weeks apart with approximately 10 »g of recombinant 12D3 antigen (using the pUC19D3 construct) as a water-in-oil-emulsion in 1 ml FCA by subcutaneous injection. These animals were then challenged with 1 X 10⁷ virulent B. bovis Lismore strain (heterologous) four weeks after the second vaccination. Serum samples were collected from the animals in weeks 0,2,4,6,7,and 8 of the vaccination regime. All vaccinated animals had weak positive reactions by ELISA at 8 weeks while by IFA the mean titre was 1/200. Control animals were negative by ELISA and <1/50 by IFA. Upon challenge the vaccinated animals had significantly lower parasitaemias than controls on days 5 and 6 (p<0.005, day 5; p,0.05, day 6) and the mean maxima were 576/»l blood and 2860/»l blood from vaccinates and controls respectively (see Figure 11). One animal in the vaccination group and three in the control group required treatment.

Sera from vaccinated animals immunoblotted both the recombinant and native 12D3 antigens.

### pGEX-3X

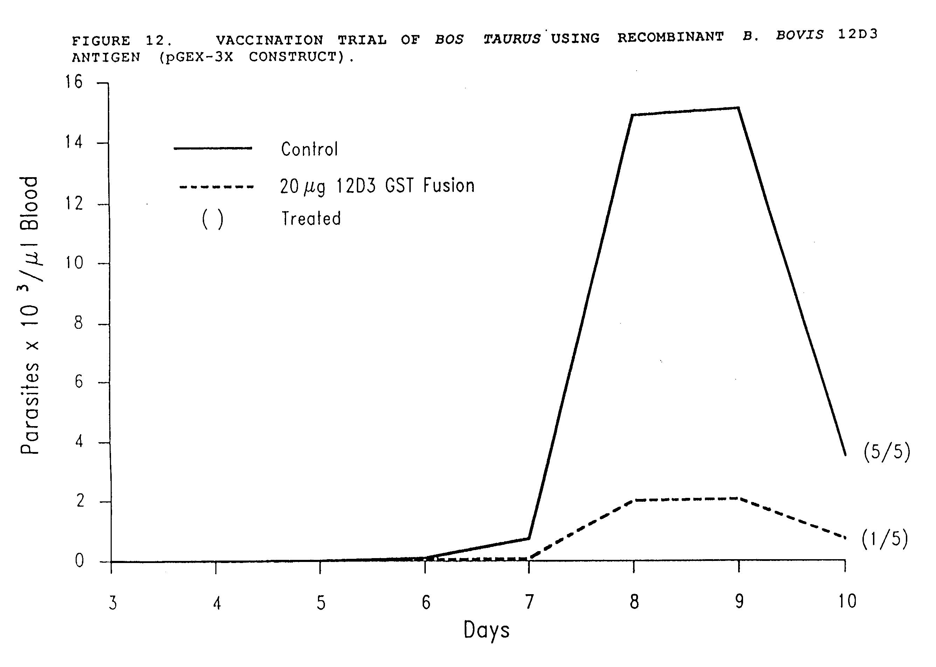

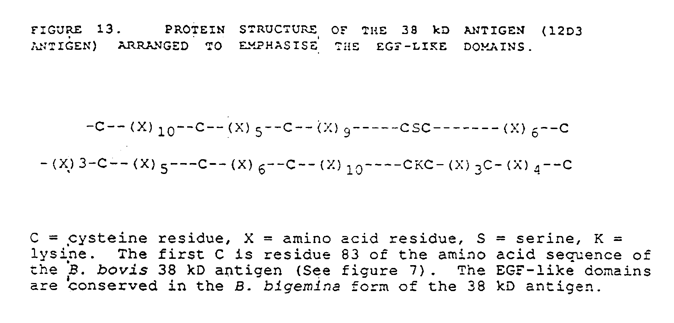

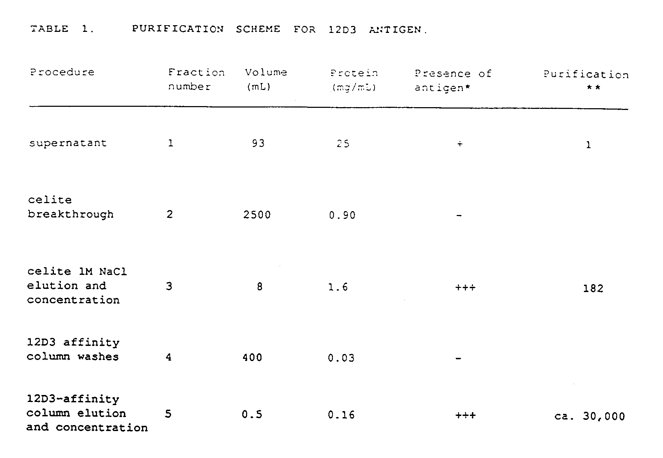

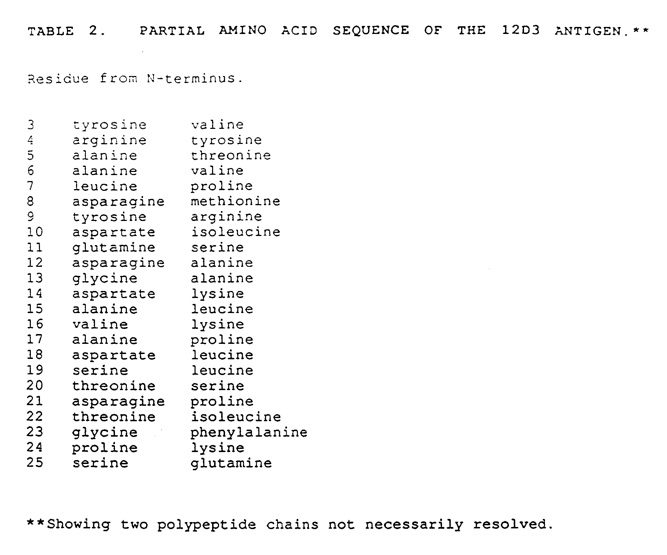

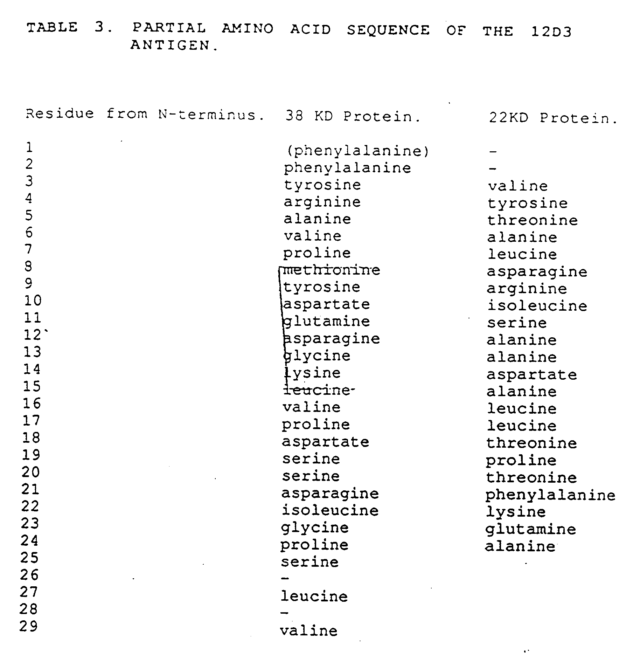

Another experiment saw the vaccination of two groups of five animals with 20 »g of antigen produced in the pGEX-3X vector) as water-in-oil emulsions in Freunds Complete Adjuvant. Two vaccinations were given using followed by challenge as above. The results indicated not only a significant delay in the development of parasitaemia (Figure 12) but a marked reduction in the levels of parasitaemia over the majority of the course of the disease. The mean maximum parasitaemias on day 9 were 15060/»l blood for controls and 2060/»l blood for the vaccinated group. The parasitaemias for the vaccinated group were significantly different from the controls, (p=0.009). All the controls were treated compared with only 1 of 5 vaccinates. It was evident that even when the recombinant antigen is denatured by SDS it is capable of producing a good level of protection against infection.

The probable existence of many disulfide bonds together with the fact that the protein is secreted and processed and contains an N-glycosylation site would make it difficult to express the antigen fully in the native state in Escherichia coli. Nevertheless, the antigen as expressed and prepared from E. coli gave a good level of protection in the experiments described above and would be suitable for inclusion in a vaccine against B. bovis. It is likely that a form of the recombinant antigen could be obtained using insect tissue culture or related systems which would be more correctly expressed and capable of producing a better protective response. The cDNA as cloned in this document would be suitable for such experiments.

The invention also includes within its scope sequences substantially homologous thereto (i.e. sequences having greater than 40% homology over a length of 100 nucleotides or longer in the case of a DNA sequence and sequences having greater than 40% homology over a length of 30 amino acids or greater in the case of a protein). The term "substantially homologous thereto" may also include within its scope DNA sequences showing cross hybridization with the 12AD3 cDNA under standard hybridization conditions.

The antigens of the invention are also useful in this invention when used as a vaccine to be protective against babesiosis particularly against homologous challenge (challenge by the same strain or isolate of Babesia) as well as heterologous challenge (challenge by different strains or isolates of Babesia). The antigens may also be protective not only against B bovis but also B. ovis, B bigemina, B canis and other strains of Babesia.

It is also noted, having regard to the foregoing, that the 38,000 kD sub-unit as described above is always associated with a further polypeptide of 22,000 kD in the native form and it is not clear whether the 22,000 kD component is a further sub-unit or chain or a bovine contaminant.

## Claims (Claims for the following Contracting State(s): AT, BE, CH, LI, DE, DK, FR, GB, IT, LU, NL, SE)

1. An antigen inducing protective immunity against homologous or heterologous challenge with *Babesia* of cattle derived from babesia infected erythrocytes and which binds to a monoclonal antibody derived from hybridoma MAbA12D3 lodged at the European Collection of Animal Cell Cultures under accession number 89072701.

2. An antigen inducing protective immunity against homologous or haterologous challenge with *Babesia* having an amino acid sequence substantially homologous with the amino acid sequence shown in FIG 7.

3. An antigen as claimed in claim 2 which is derived from a gene showing a DNA sequence substantially homologous with the sequence shown in FIG 9.

4. An antigen as claimed in claim 2 having a partial amino acid sequence in relation to a 38 kD sub-unit as shown in Table 3.

5. An antigen as claimed in any one of claims 2-4 obtained as a genetically engineered polypeptide in the form of a fusion protein.

6. An antigen as claimed in claim 5 wherein the antigen is coupled to B-galactosidase.

7. An antigen as claimed in claim 5 herein the antigen is coupled to glutathione-S-transferase.

8. An antigen as claimed in claim 2 which is derived from a gene showing a DNA sequence substantially homologous with the sequence shown in FIG 6.

9. An antigen as claimed in claim 2 having a molecular weight of 38,000 and having two EGF-like domains as shown in FIG 13.

10. An antigen inducing protective immunity against homologous or heterologous challenge with *Babesia* having the following properties:
(i) one sub-unit having a molecular weight of 38,000;
(ii) an N terminus end of the 38,000 molecular weight sub-unit including the sequence Methionine-Tyrosine-Aspartate-Glutamine-Asparagine-Glycine-Lysine; and
(iii) another polypeptide having a molecular weight of 22,000 which is bound or associated with the sub-unit of molecular weight 38,000.

11. A monoclonal antibody derived from hybridoma MAbA12D3 lodged at the European Collection of Animal Cell Cultures under accession number 89072701.

12. A DNA sequence encoding for an antigen as claimed in claim 2 and having the structure shown FIG 6 and a sequence substantially homologous thereto

13. A DNA sequence encoding for an antigen as claimed in claim 2 and having the structure shown in FIG 9 and a sequence substantially homologous thereto.

14. A vaccine containing the antigen of any one of claims 1-10 in combination with an adjuvant.

15. A test kit which may be used for detection of babesiosis including a monoclonal antibody as claimed in claim 11.

16. A vaccine containing an antigen derived from a DNA sequence as claimed in claim 12 or 13 in combination with an adjuvant.

17. A method for the preparation of an antigen as claimed in claim 2 including the steps of:
(i) purifying an antigenic fraction of *Babesia*;
(ii) sequencing the N-terminus of the purified antigenic fraction from step (i);
(iii) preparing one or more synthetic oligonucleotides based on the sequence obtained from step (ii);
(iv) preparing cDNA library from m-RNA derived from *Babesia*;
(v) screening the cDNA library using the synthetic oligonucleotide prepared from step (iii) as a probe;
(vi) purifying positive clone(s) and subcloning the cDNA into an expression vector; and
(vii) expressing the clone(s) in the expression vector and obtaining the resultant antigen.

18. A process as claimed in claim 17 wherein the antigenic fraction is purified by isolation of a corresponding monoclonal antibody from a lysate of *Babesia* infected erythrocytes and subsequent purification of the monoclonal antibody by attachment of the monoclonal antibody to an immobilised support and subsequent elution at a pH of between 7.0-9.5 whereafter the monoclonal antibody is immobilised or insolubilized and coupled to the antigenic fraction prepared from *Babesia* infected erythrocytes which antigenic fraction is subsequently eluted therefrom.

19. A process as claimed in claim 18 wherein the monoclonal antibody is eluted at a pH of 8.6 from said immobilised support.

20. A process as claimed in claim 19 wherein the antigenic fraction has a pH of between 6.0-9.5 before and after contact with the monoclonal antibody.

21. A process as claimed in claim 20 wherein the pH of the antigenic fraction is 7.4.

22. A method as claimed in any one of claims 18-21 wherein a purified antigen obtained from the antigenic extract has a sub-unit of molecular weight of 38,000.

23. A method as claimed in claim 22 wherein sequencing of the N-terminal amino acid end includes a sequence Methionine-Tyrosine-Aspartate-Glutamine-Asparagine-Glycine-Lysine.

## Claims (Claims for the following Contracting State(s): ES, GR)

1. A method for the preparation of an antigen inducing protective immunity against homologous or heterologous challenge with Babesia having an amino acid sequence substantially homologous with the amino acid sequence shown in Fig.7 including the steps of:
(i) purifying an antigenic fraction of Babesia;
(ii) sequencing the N-terminus of the purified antigenic fraction from step (i);
(iii) preparing one or more synthetic oligonucleotides based on the sequence obtained from step (ii);
(iv) preparing cDNA library from m-RNA derived from Babesia;
(v) screening the cDNA library using the synthetic oligonucleotide prepared from step (iii) as a probe;
(vi) purifying positive clone(s) and subcloning the cDNA into an expression vector; and
(vii) expressing the clone(s) in the expression vector and obtaining the resultant antigen.

2. A method according to claim 1 wherein said antigen is derived from a gene showing a DNA sequence substantially homologous with the sequence shown in Fig.9.

3. A method according to claim 1 wherein said antigen has a partial amino acid sequence in relation to a 38 kD sub-unit as shown in Table 3.

4. A method according to any one of claims 1-3 wherein said antigen is obtained as a genetically engineered polypeptide in the form of a fusion protein.

5. A method according to claim 4 wherein the antigen is coupled to B-galactosidase.

6. A method according to claim 4 wherein said antigen is coupled to glutathione-S-transferase.

7. A method according to claim 1 wherein said antigen is derived from a gene showing a DNA sequence substantially homologous with the sequence shown in Fig.6.

8. A method according to claim 1 wherein said antigen has a molecular weight of 38,000 and having two EGF-like domains as shown in Fig.13.

9. A method according to claim 1 wherein said antigen has the following properties:
(i) one sub-unit having a molecular weight of 38,000;
(ii) an N terminus end of the 38,000 molecular weight sub-unit including the sequence Methionine-Tyrosine-Aspartate-Glutamine-Asparagine-Glycine-Lysine; and
(iii) another polypeptide having a molecular weight of 22,000 which is bound or associated with the sub-unit of molecular weight 38,000.

10. A process as claimed in claim 1 wherein the antigenic fraction is purified by isolation of a corresponding monoclonal antibody from a lysate of Babesia infected erythrocytes and subsequent purification of the monoclonal antibody by attachment of the monoclonal antibody to an immobilised support and subsequent elution at a pH of between 7.0-9.5 whereafter the monoclonal antibody is immobilised or insolubilized and coupled to the antigenic fraction prepared from Babesia infected erythrocytes which antigenic fraction is subsequently eluted therefrom.

11. A process as claimed in claim 10 wherein the monoclonal antibody is eluted at a pH of 8.6 from said immobilised support.

12. A process as claimed in claim 11 wherein the antigenic fraction has a pH of between 6.0-9.5 before and after contact with the monoclonal antibody.

13. A process as claimed in claim 12 wherein the pH of the antigen fraction is 7.4.

14. A method as claimed in any one of claims 10-13 wherein a purified antigen obtained from the antigenic extract has a sub-unit of molecular weight of 38,000.

15. A method as claimed in claim 14 wherein sequencing of the N-terminal amino acid end includes a sequence Methionine-Tyrosine-Aspartate-Glutamine-Asparagine-Glycine-Lysine.

16. A method according to claim 10 wherein said antigen induces protective immunity against homologous or heterologous challenge with Babesia of cattle derived from babesia infected erythrocytes and which binds to a monoclonal antibody derived from hybridoma MAbA12D3 lodged at the European Collection of Animal Cell Cultures under accession number 89072701.

17. A method for the production of a vaccine containing the antigen of any one of claims 1-16 wherein said antigen is combined with an adjuvant.

18. A method for the production of a monoclonal antibody wherein said monoclonal antibody is obtainable from hybridoma MAbA12D3 lodged at the European Collection of Animal Cell Cultures under accession number 89072701.

19. A test kit which may be used for the detection of babesiosis including a monoclonal antibody as obtainable in claim 18.

## Patentansprüche (Patentansprüche für folgende(n) Vertragsstaat(en): AT, BE, CH, LI, DE, DK, FR, GB, IT, LU, NL, SE)

1. Ein Antigen, das protektive Immunität gegen homologe oder heterologe Exposition mit Babesia bei Rindern induziert, gewonnen aus Babesia-infizierten Erythrocyten, und das an einen monoklonalen Antikörper, gewonnen aus Hybridoma MAbA12D3, hinterlegt bei der European Collection of Animal Cell Cultures unter der Hinterlegungsnummer 89072701, bindet.

2. Antigen, das eine protektive Immunität gegen homologe oder heterologe Exposition mit Babesia induziert, mit einer Aminosäuresequenz, die im wesentlichen homolog mit der Aminosäuresequenz, wie in Fig. 7 gezeigt, ist.

3. Antigen nach Anspruch 2, das aus einem Gen gewonnen wird, das eine zur Sequenz in Fig. 9 im wesentlichen homologe DNA-Sequenz zeigt.

4. Antigen nach Anspruch 2, mit einer in Bezug auf eine 38-kD-Untereinheit wie in Tabelle 3 gezeigt partiellen Aminosäuresequenz.

5. Antigen nach einem der Ansprüche 2 bis 4, erhalten als gentechnisch hergestelltes Polypeptid in Form eines Fusionsproteins.

6. Antigen nach Anspruch 5, worin das Antigen an eine B-Galactosidase gekoppelt ist.

7. Antigen nach Anspruch 5, worin das Antigen an Glutathion-S-Transferase gekoppelt ist.

8. Antigen nach Anspruch 2, das aus einem Gen gewonnen wird, das die mit der Sequenz in Fig. 6 im wesentlichen homologe DNA-Sequenz zeigt.

9. Antigen nach Anspruch 2, mit dem Molekulargewicht von 38000 und zwei EGF-ähnlichen Domänen, wie in Fig. 13 gezeigt.

10. Antigen, das eine protektive Immunität gegen homologe oder heterologe Exposition mit Babesia induziert, mit den folgenden Eigenschaften:
(i) eine Untereinheit hat ein Molekulargewicht von 38000;
(ii) ein N-terminales Ende der Untereinheit mit dem Molekulargewicht von 38000 umfaßt die Sequenz Methionin-Tyrosin-Asparaginsäure-Glutamin-Asparagin-Glycin-Lysin; und
(ii) ein weiteres Polypeptid mit einem Molekulargewicht von 22000, das an die Untereinheit mit dem Molekulargewicht von 38000 gebunden oder assoziiert ist.

11. Monoklonaler Antikörper, gewonnen aus Hybridoma MAbA12D3, hinterlegt bei der European Collection of Animal Cell Cultures unter der Hinterlegungsnummer 89072701.

12. DNA-Sequenz, kodierend für ein Antigen nach Anspruch 2 und mit der in Fig. 6 gezeigten Struktur, und eine Sequenz, die im wesentlichen homolog hierzu ist.

13. DNA-Sequenz, kodierend für ein Antigen nach Anspruch 2 und mit einer Struktur wie in Fig. 9 gezeigt, und eine Sequenz, die im wesentlichen homolog hierzu ist.

14. Impfstoff, enthaltend das Antigen nach einem der Ansprüche 1 bis 10 in Kombination mit einem Adjuvanz.

15. Testkit, das zur Detektion auf Babesiose verwendet werden kann, umfassend einen monoklonalen Antikörper gemäß Anspruch 11.

16. Impfstoff, enthaltend ein Antigen, das aus einer DNA-Sequenz nach Anspruch 12 oder 13 abgeleitet ist, in Kombination mit einem Adjuvanz.

17. Verfahren zur Herstellung eines Antigens nach Anspruch 2, umfassend die Schritte:
(i) Reinigen einer antigenischen Fraktion von Babesia;
(ii) Sequenzieren des N-Terminus der gereinigten antigenen Fraktion aus Schritt (i);
(iii) Herstellen von einem oder mehreren synthetischen Oligonukleotiden beruhend auf der Sequenz erhalten aus Schritt aus (ii);
(iv) Herstellen einer cDNA-Bank aus m-RNA abgeleitet aus Babesia;
(v) Screenen der cDNA-Bank unter Verwendung des in Schritt (iii) hergestellten synthetischen Oligonukleotids als Sonde;
(vi) Reinigen des (der) positiven Klons(e) und Subklonieren der cDNA in einem Expressionsvektor; und
(vii) Exprimieren der (des) Klone(s) im Expressionsvektor und Gewinnen des resultierenden Antigens.

18. Verfahren nach Anspruch 17, worin die antigene Fraktion durch Isolierung eines entsprechenden monoklonalen Antikörpers gewonnen wird aus einem Lysat von Babesia-infizierten Erythrocyten und anschließender Reinigung des monoklonalen Antikörpers durch Anheftung des monoklonalen Antikörpers an einen immobilisierten Träger und anschließender Elution bei einem pH von zwischen 7,0 bis 9,5, worauf der monoklonale Antikörper immobilisiert oder unlöslich gemacht wird und an eine antigene Fraktion, hergestellt aus Babesia-infizierten Erythrocyten, gekoppelt wird, wobei die antigene Fraktion anschließend hiervon eluiert wird.

19. Verfahren nach Anspruch 18, worin der monoklonale Antikörper bei einem pH von 8,6 von dem immobilisierten Träger eluiert wird.

20. Verfahren nach Anspruch 19, worin die antigene Fraktion einen pH von 6,0 bis 9,5 vor und nach dem Kontakt mit dem monoklonalen Antikörper hat.

21. Verfahren nach Anspruch 20, worin der pH der antigenen Fraktion 7,4 ist.

22. Verfahren nach einem der Ansprüche 18 bis 21, worin ein gereinigtes Antigen, erhalten aus dem Antigenextrakt, eine Untereinheit mit einem Molekulargewicht von 38000 hat.

23. Verfahren nach Anspruch 22, worin das Sequenzieren des N-terminalen Aminosäuresequenz-Endes eine Sequenz Methionin-Tyrosin-Asparaginsäure-Glutamin-Asparagin-Glycin-Lysin umfaßt.

## Patentansprüche (Patentansprüche für folgende(n) Vertragsstaat(en): ES, GR)

1. Verfahren zur Herstellung eines Antigens, das eine protektive Immunität gegen homologe oder heterologe Exposition mit Babesia induziert, mit einer Aminosäuresequenz, die im wesentlichen homolog mit der Aminosäuresequenz, wie in Fig. 7 gezeigt, ist, umfassend die Schritte:
(i) Reinigen einer antigenischen Fraktion von Babesia;
(ii) Sequenzieren des N-Terminus der gereinigten antigenen Fraktion aus Schritt (i);
(iii) Herstellen von einem oder mehreren synthetischen Oligonukleotiden beruhend auf der Sequenz erhalten aus Schritt aus (ii);
(iv) Herstellen einer cDNA-Bank aus m-RNA abgeleitet aus Babesia;
(v) Screenen der cDNA-Bank unter Verwendung des in Schritt (iii) hergestellten synthetischen Oligonukleotids als Sonde;
(vi) Reinigen des (der) positiven Klons(e) und Subklonieren der cDNA in einem Expressionsvektor; und
(vii) Exprimieren der (des) Klone(s) im Expressionsvektor und Gewinnen des resultierenden Antigens.

2. Verfahren nach Anspruch 1, worin das Antigen von einem Gen mit einer DNA-Sequenz abgeleitet ist, die mit der in Fig. 9 gezeigten Sequenz im wesentlichen homolog ist.

3. Verfahren nach Anspruch 1, worin das Antigen eine partielle Aminosäuresequenz in Bezug auf eine 38-kD-Untereinheit, wie Tabelle 3 gezeigt, hat.

4. Verfahren nach einem der Ansprüche 1 bis 3, worin das Antigen erhalten wird als gentechnisch hergestelltes Polypeptid in Form eines Fusionsproteins.

5. Verfahren nach Anspruch 4, worin das Antigen an B-Galactosidase gekoppelt ist.

6. Verfahren nach Anspruch 4, worin das Antigen ein Glutathion-S-Transferase gekoppelt ist.

7. Verfahren nach Anspruch 1, worin das Antigen von einem Gen stammt, das eine DNA-Sequenz zeigt, die im wesentlichen homolog mit der in Fig. 6 gezeigten Sequenz ist.

8. Verfahren nach Anspruch 1, worin das Antigen ein Molekulargewicht von 38000 hat und zwei EGF-ähnliche Domänen, wie in Fig. 13 gezeigt, hat.

9. Verfahren nach Anspruch 1, worin das Antigen die folgenden Eigenschaften hat:
(i) eine Untereinheit hat ein Molekulargewicht von 38000;
(ii) ein N-terminales Ende der Untereinheit mit dem Molekulargewicht von 38000 umfaßt die Sequenz Methionin-Tyrosin-Asparaginsäure-Glutatamin-Asparagin-Glycin-Lysin; und
(ii) ein weiteres Polypeptid mit einem Molekulargewicht von 22000, das an die Untereinheit mit dem Molekulargewicht von 38000 gebunden oder assoziiert ist.

10. Verfahren nach Anspruch 1, worin die antigene Fraktion durch Isolierung eines entsprechenden monoklonalen Antikörpers gewonnen wird aus einem Lysat von Babesia-infizierten Erythrocyten und anschließender Reinigung des monoklonalen Antikörpers durch Anheftung des monoklonalen Antikörpers an einen immobilisierten Träger und anschließender Elution bei einem pH von zwischen 7,0 bis 9,5, worauf der monoklonale Antikörper immobilisiert oder unlöslich gemacht wird und an eine antigene Fraktion, hergestellt aus Babesia-infizierten Erythrocyten, gekoppelt wird, wobei die antigene Fraktion anschließend hiervon eluiert wird.

11. Verfahren nach Anspruch 10, worin der monoklonale Antikörper bei einem pH von 8,6 von dem immobilisierten Träger eluiert wird.

12. Verfahren nach Anspruch 11, worin die antigene Fraktion einen pH von 6,0 bis 9,5 vor und nach dem Kontakt mit dem monoklonalen Antikörper hat.

13. Verfahren nach Anspruch 12, worin der pH der antigenen Fraktion 7,4 ist.

14. Verfahren nach einem der Ansprüche 10 bis 13, worin ein gereinigtes Antigen, erhalten aus dem Antigenextrakt, eine Untereinheit mit einem Molekulargewicht von 38000 hat.

15. Verfahren nach Anspruch 14, worin das Sequenzieren des N-terminalen Aminosäuresequenz-Endes eine Sequenz Methionin-Tyrosin-Asparaginsäure-Glutamin-Asparagin-Glycin-Lysin umfaßt.

16. Verfahren nach Anspruch 10, worin das Antigen eine protektive Immunität gegen homologe oder heterologe Exposition mit Babesia bei Rindern induziert, gewonnen aus Babesia-infizierten Erythrocyten, und das an einen monoklonalen Antikörper, gewonnen aus Hybridoma MAbA12D3, hinterlegt bei der European Collection of Animal Cell Cultures unter der Hinterlegungsnummer 89072701.

17. Verfahren zur Herstellung eines Impfstoffes, enthaltend das Antigen nach einem der Ansprüche 1 bis 16, worin das Antigen mit einem Adjuvanz kombiniert ist.

18. Verfahren zur Herstellung eines monokonalen Antikörpers, worin der monoklonale Antikörper aus Hybridoma MAbA12D3, hinterlegt bei der European Collection of Animal Cell Cultures unter der Hinterlegungsnummer 89072701, erhältlich ist.

19. Testkit, das zum Nachweis von Babesiose verwendet werden kann, umfassend einen monoklonalen Antikörper, erhältlich gemäß Anspruch 18.

## Revendications (Revendications pour l'(les) Etat(s) contractant(s) suivant(s): AT, BE, CH, LI, DE, DK, FR, GB, IT, LU, NL, SE)

1. Antigène induisant une immunité protectrice vis-à-vis d'une provocation homologue ou hétérologue par *Babesia* des bovidés, qui dérive d'érythrocytes infectés par des babésies, et qui se fixe à un anticorps monoclonal qui dérive de l'hybridome MAbA12D3, déposé auprès de l'European Collection of Animal Cell Cultures sous le numéro d'enregistrement 89072701.

2. Antigène induisant une immunité protectrice vis-à-vis d'une provocation homologue ou hétérologue par *Babesia*, comportant une séquence d'acides aminés essentiellement homologue de la séquence d'acides aminés présentée sur la Figure 7.

3. Antigène selon la revendication 2, qui dérive d'un gène présentant une séquence d'ADN essentiellement homologue de la séquence présentée sur la Figure 9.

4. Antigène selon la revendication 2, comportant une séquence partielle d'acides aminés en liaison avec une sous-unité de 38 kD telle que consignée dans le Tableau 3.

5. Antigène selon l'une quelconque des revendications 2 à 4, obtenue sous forme d'une protéine de fusion, en tant que polypeptide obtenu par génie génétique.

6. Antigène selon la revendication 5, dans lequel l'antigène est couplé à la β-galactosidase.

7. Antigène selon la revendication 5, dans lequel l'antigène est couplé à la glutathione-S-transférase.

8. Antigène selon la revendication 2, qui dérive d'un gène présentant une séquence d'ADN essentiellement homologue de la séquence présentée sur la Figure 6.

9. Antigène selon la revendication 2, ayant une masse moléculaire de 38 000 et possédant deux domaines de type EGF, tels que présentés sur la Figure 13.

10. Antigène induisant une immunité protectrice vis-à-vis d'une provocation homologue ou hétérologue par *Babesia*, présentant les propriétés suivantes :
(i) une sous-unité ayant une masse moléculaire de 38 000 ;
(ii) un site N-terminal de la sous-unité ayant une masse moléculaire de 38 000, comprenant la séquence méthionine-tyrosine-aspartate-glutamine-asparagine-glycine-lysine ; et
(iii) un autre polypeptide ayant une masse moléculaire de 22 000, qui est lié ou associé à la sous-unité ayant une masse moléculaire de 38 000.

11. Anticorps monoclonal, qui dérive de l'hybridome MAbA12D3, déposé auprès de l'European Collection of Animal Cell Cultures sous le numéro d'enregistrement 89072701.

12. Séquence d'ADN codant pour un antigène selon la revendication 2 et ayant la structure présentée sur la Figure 6, et une séquence qui lui est essentiellement homologue.

13. Séquence d'ADN codant pour un antigène selon la revendication 2 et ayant la structure présentée sur la Figure 9, et une séquence qui lui est essentiellement homologue.

14. Vaccin contenant l'antigène selon l'une quelconque des revendications 1 à 10, en combinaison avec un adjuvant.

15. Trousse d'essai pouvant être utilisée pour détecter une babésiose, comprenant un anticorps monoclonal selon la revendication 11.

16. Vaccin contenant un antigène dérivant d'une séquence d'ADN selon la revendication 12 ou 13 en combinaison avec un adjuvant.

17. Procédé pour préparer un antigène selon la revendication 2, qui comprend les étapes consistant :
(i) à purifier une fraction antigénique de *Babesia ;*
(ii) à séquencer le site N-terminal de la fraction antigénique purifiée obtenue dans l'étape (i) ;
(iii) à préparer un ou plusieurs oligonucléotides synthétiques sur la base de la séquence obtenue dans l'étape (ii) ;
(iv) à préparer une banque d'ADNc à partir de l'ARN-m qui dérive de *Babesia ;*
(v) à cribler la banque d'ADNc en utilisant comme sonde l'oligonucléotide synthétique préparé dans l'étape (iii) ;
(vi) à purifier le ou les clones positifs et à sous-cloner l'ADNc dans un vecteur d'expression ; et
(vii) à exprimer le ou les clones dans le vecteur d'expression et à obtenir l'antigène final.

18. Procédé selon la revendication 17, dans lequel on purifie la fraction antigénique en isolant un anticorps monoclonal correspondant d'un lysat d'érythrocytes infectés par *Babesia*, puis en purifiant l'anticorps monoclonal en fixant l'anticorps monoclonal à un support immobilisé, puis en éluant à pH compris entre 7,0 et 9,5, puis on immobilise ou insolubilise l'anticorps monoclonal et on le couple à la fraction antigénique préparée à partir d'érythrocytes infectés par *Babesia*, la fraction antigénique en étant ensuite séparée par élution.

19. Procédé selon la revendication 18, dans lequel l'anticorps monoclonal est élué du support immobilisé à pH 8,6.

20. Procédé selon la revendication 19, dans lequel la fraction antigénique a un pH compris entre 6,0 et 9,5 avant et après contact avec l'anticorps monoclonal.

21. Procédé selon la revendication 20, dans lequel le pH de la fraction antigénique est de 7,4.

22. Procédé selon l'une quelconque des revendications 18 à 21, dans lequel un antigène purifié obtenu à partir de l'extrait antigénique a une sous-unité dont la masse moléculaire est de 38 000.

23. Procédé selon la revendication 22, dans lequel le séquençage de l'extrémité d'acides aminés N-terminal comprend une séquence méthionine-tyrosine-aspartate-glutamine-asparagine-glycine-lysine.

## Revendications (Revendications pour l'(les) Etat(s) contractant(s) suivant(s): ES, GR)

1. Procédé pour préparer un antigène induisant une immunité protectrice contre une provocation homologue ou hétérologue par *Babesia*, ayant une séquence d'acides aminés essentiellement homologue de la séquence d'acides aminés présentée sur la Figure 7, et comprenant les étapes consistant :
(i) à purifier une fraction antigénique de *Babesia ;*
(ii) à séquencer le site N-terminal de la fraction antigénique purifiée obtenue dans l'étape (i) ;
(iii) à préparer un ou plusieurs oligonucléotides synthétiques sur la base de la séquence obtenue dans l'étape (ii) ;
(iv) à préparer une banque d'ADNc à partir de l'ARN-m qui dérive de *Babesia* ;
(v) à cribler la banque d'ADNc en utilisant comme sonde l'oligonucléotide synthétique préparé dans l'étape (iii) ;
(vi) à purifier le ou les clones positifs et à sous-cloner l'ADNc dans un vecteur d'expression ; et
(vii) à exprimer le ou les clones dans le vecteur d'expression et à obtenir l'antigène final.

2. Procédé selon la revendication 1, dans lequel l'antigène dérive d'un gène présentant une séquence d'ADN essentiellement homologue de la séquence présentée sur la Figure 9.

3. Procédé selon la revendication 1, dans lequel l'antigène a une séquence partielle d'acides aminés en liaison avec une sous-unité de 38 kD telle que consignée dans le Tableau 3.

4. Procédé selon l'une quelconque des revendications 1 à 3, dans lequel l'antigène est obtenu sous forme d'une protéine de fusion en tant que polypeptide obtenue par génie génétique.

5. Procédé selon la revendication 4, dans lequel l'antigène est couplé à la β-galactosidase.

6. Procédé selon la revendication 4, dans lequel l'antigène est couplé à la glutathione-S-transférase.

7. Procédé selon la revendication 1, dans laquelle l'antigène dérive d'un gêne présentant une séquence d'ADN essentiellement homologue de la séquence présentée sur la Figure 6.

8. Procédé selon la revendication 1, dans lequel l'antigène a une masse moléculaire de 38 000 et possède deux domaines de type EGF, comme on le voit sur la Figure 13.

9. Procédé selon la revendication 1, dans lequel l'antigène a les propriétés suivantes :
(i) une sous-unité ayant une masse moléculaire de 38 000 ;
(ii) un site N-terminal de la sous-unité ayant une masse moléculaire de 38 000, comprenant la séquence méthionine-tyrosine-aspartate-glutamine-asparagine-glycine-lysine ; et
(iii) un autre polypeptide ayant une masse moléculaire de 22 000, qui est lié ou associé à la sous-unité ayant une masse moléculaire de 38 000.

10. Procédé selon la revendication 1, dans lequel on purifie la fraction antigénique en isolant un anticorps monoclonal correspondant d'un lysat d'érythrocytes infectés par *Babesia*, puis en purifiant l'anticorps monoclonal en fixant l'anticorps monoclonal à un support immobilisé, puis en éluant à pH compris entre 7,0 et 9,5, puis on immobilise ou insolubilise l'anticorps monoclonal et on le couple à la fraction antigénique préparée à partir d'érythrocytes infectés par *Babesia*, la fraction antigénique en étant ensuite séparée par élution.

11. Procédé selon la revendication 10, dans lequel l'anticorps monoclonal est élué du support immobilisé à pH 8,6.

12. Procédé selon la revendication 11, dans lequel la fraction antigénique a un pH compris entre 6,0 et 9,5 avant et après contact avec l'anticorps monoclonal.

13. Procédé selon la revendication 12, dans lequel le pH de la fraction antigénique est de 7,4.

14. Procédé selon l'une quelconque des revendications 10 à 13, dans lequel un antigène purifié obtenu à partir de l'extrait antigénique a une sous-unité dont la masse moléculaire est de 38 000.

15. Procédé selon la revendication 14, dans lequel le séquençage de l'extrémité d'acides aminés N-terminal comprend une séquence méthionine-tyrosine-aspartate-glutamine-asparagine-glycine-lysine.

16. Procédé selon la revendication 10, dans lequel l'antigène induit une immunité protectrice à l'encontre d'une provocation homologue ou hétérologue par *Babesia* des bovidés, dérivant d'érythrocytes infectés par *Babesia*, et qui se fixe à un anticorps monoclonal dérivant de l'hybridome MAbA12D3, déposé auprès de l'European Collection of Animal Cell Cultures sous le numéro d'enregistrement 89072701.

17. Procédé pour produire un vaccin contenant l'antigène selon l'une quelconque des revendications 1 à 16, où l'antigène est combiné à un adjuvant.

18. Procédé pour produire un anticorps monoclonal, dans lequel l'anticorps monoclonal peut être obtenu à partir de l'hybridome MAbA12D3, déposé auprès de l'European Collection of Animal Cell Cultures sous le numéro d'enregistrement 89072701.

19. Trousse d'essai pouvant être utilisée pour détecter la babésiose, comprenant un anticorps monoclonal tel qu'on peut l'obtenir dans la revendication 18.
